# EUROPEAN PATENT SPECIFICATION

(11) **EP 1 692 169 B1**
(45) Date of publication and mention of the grant of the patent: **20.08.2008**
(21) Application number: 04799975.0
(22) Date of filing: 01.11.2004
(51) Int. Cl.: C12N 9/12

(54) **DDR2 PROTEIN WITH ACTIVATED KINASE ACTIVITY AND PREPARATION METHOD THEREOF**
DDR2-PROTEIN MIT AKTIVIERTER KINASEAKTIVITÄT UND VERFAHREN ZU DESSEN HERSTELLUNG
PROTEINE DU DDR2 A ACTIVITE DE KINASE ACTIVEE ET PROCEDE DE PREPARATION DE CELLE-CI

(30) Priority: 31.10.2003 KR 2003076967
(43) Date of publication of application: 23.08.2006
(73) Proprietor: Korea Institute of Science and Technology, Seoul 136-791 (KR); Jeil Pharmaceutical Co., Ltd., Seoul 137-041 (KR)
(72) Inventor: YANG, Beom-Seok, Seoul 140-131 (KR); PARK, Sung-Dae, Seoul 137-030 (KR)
(74) Representative: Daniels, Jeffrey Nicholas
(86) International application number: PCT/KR2004/002784
(87) International publication number: WO 2005/042577

(56) References cited:
- WO-A2-98/34954
- K. IKEDA ET AL.: "Discoidin domain receptor 2 interacts with Src and Shc following its activation by type I collagen" THE JOURNAL OF BIOLOGICAL CHEMISTRY, vol. 277, no. 21, 24 May 2002 (2002-05-24), pages 19206-19212, XP002422281
- W. VOGEL: "Discoidin domain receptors: structural relations and functional implications" FASEB, vol. suplemental, 1999, pages S77-S82, XP002422282
- VOGEL W ET AL: "The discoidin domain receptor tyrosine kinases are activated by collagen" MOLECULAR CELL, CELL PRESS, CAMBRIDGE, MA, US, December 1997 (1997-12), XP002071992 ISSN: 1097-2765
- K. YANG ET AL: "Tyrosine 740 phosphorylation of discoidin domain receptor 2 by Src stimulates intramolecular autophosphorylation and Shc signaling complex formation" THE JOURNAL OF BIOLOGICAL CHEMISTRY, vol. 280, no. 47, 25 November 2005 (2005-11-25), pages 39058-39066, XP002422283
- LEITINGER B. ET AL.: 'Molecular analysis of collagen binding by the human discoidin domain receptors, DDR1 and DDR2' J. BIOL. CHEM. vol. 278, no. 19, 09 May 2003, pages 16761 - 16769, XP003010388
- IKEDA K. ET AL.: 'Discoidin domain receptor 2 interacts with Src and Shc following its activation by type I collagen' J. BIOL. CHEM. vol. 277, no. 21, 24 May 2002, pages 19206 - 19212, XP002422281
- OLASO E. ET AL.: 'DDR2 receptor promotes MMP-2-mediated proliferation and invasion by hepatic stellate cells' J. CLIN. INVEST. vol. 108, no. 9, November 2001, pages 1369 - 1378, XP002401680

## Description

### Technical Field

The present invention relates to a protein containing a modified DDR (Discoidin Domain Receptor) 2 cytosolic tyrosine kinase domain having an increased autophosphorylation and tyrosine kinase activity; a method for preparing a large amount of a protein containing DDR2 cytosolic tyrosine kinase domain, having an increased autophosphorylation and tyrosine kinase activity by inducing phosphorylations of tyrosines by a co-expression with Src or Src related proteins in host cells, or by H₂O₂ processing of host cells, or a site directed mutation modifying at least one of tyrosines to other amino acid; and a use thereof as a target material in developing medical drugs for treating a disease caused by an excessively activated DDR2 autophosphorylation and tyrosine kinase activity.

### Background Art

One of the ways that cells recognize an external stimulus is to recognize through receptor tyrosine kinase family present on cell membrane. Receptor tyrosine kinase family protein is a trans-membrane protein consisting of an extra-cellular domain exposed to outside of cell, to which a specific ligand binds; an intracellular domain exposed to cytoplasm of cell inside, which transfers an activating signal of the receptor activated by the ligand binding into the inside of cell; and transmembrane domain. The intra-cellular domain of receptor tyrosine kinase family protein has a tyrosine kinase activity domain and when a specific ligand binds to its extra-cellular domain, its tyrosine kinase activity is activated to phosphorylate tyrosines in its cytosolic domain. Such tyrosine phosphorylation is the most important process to transfer the signal of an external stimulus inside cells via receptor tyrosine kinase family protein.

DDR (Disooidin Domain Receptor) protein is one of receptor tyrosine kinase family having a tyrosine kinase activity domain in its cytosolic domain and the active ligand thereof is various collagens. In case of animals including the human beings, there are two types of DDR proteins, DDR1 type and DDR2 type proteins, which have similar amino acid sequences and encoded by different genes to each other.

Recently, it has been reported that the activation or the increased production of DDR2 protein relates to human intractable diseases, e.g., liver cirrhosis, rheumatism, cancer metastasis and so forth. Like other receptor tyrosine kinase proteins, when activated by ligand, the cytosolic domain of DDR2 is auto-phosphorylated by its increased tyrosine kinase activity. The activation of the tyrosine kinase activity of DDR2 protein is known to be necessary to stimulate the cell growth of liver stellate cells, fibroblasts or synovial fibroblasts from a patient with rheumatism, and simultaneously, to increase the production of fiber collagen as well as the production of protease, such as MMP-1 or MMP-2. The cells of which growth are accelerated as above, and the collagen and MMP-1 or MMP-2 produced by the cells have been known as one factor of direct causes for a tissue cirrhosis, rheumatism and/or cancer metastasis.

Src protein is one of non-receptor type tyrosine kinases. It has several homologous proteins so called src family protein where fyn, yes, lck, hck, lyn, csk, blk, etc are included. The various functions thereof in cells have been reported. In particular, the protein has been known to perform a function to increase the activity of the receptor-type tyrosine kinase, such as EGFR, PDGFR, or the like. Recently it has been reported that src tyrosine kinase activity is required for DDR2 cellular signaling.

### Disclosure of Invention

### Technical Solution

SUMMARY OF THE INVENTION

Therefore, an object of the present invention is to provide a protein containing a modified human DDR 2 cytosolic tyrosine kinase domain having an increased autophosphorylation and tyrosine kinase activity, wherein tyrosine 740, in the activation loop of the DDR2 cytosolic tyrosine kinase domain is modified by inducing a phosphorylation of tyrosine, or by mutating to phenylalanine, by a site-directed mutation.

Another object of the present invention is to provide a method for preparing a large amount of a protein containing DDR2 cytosolic tyrosine kinase domain protein having an increased autophosphorylation and tyrosine kinase activity, by phosphorylating tyrosine 740 of the activation loop of the human DDR2 cytosolic tyrosine kinase domain through co-expression together with Src protein or with Src related proteins, such as Fyn, Yes, Lck, Hck, Lyn, Csk, Blk, etc.

Another object of the present invention is to provide a method for preparing a large amount of a protein containing DDR2 cytosolic tyrosine kinase domain having an increased autophosphorylation and tyrosine kinase activity by phosphorylating tyrosine at the DDR2 cytosolic tyrosine kinase domain protein through H₂O₂ processing.

Another object of the present invention is to provide a method for preparing a large amount of a protein containing DDR2 cytosolic tyrosine kinase domain having an increased autophosphorylation and tyrosine kinase activity, by modifying tyrosine 740 of the activation loop of human DDR2 cytosolic tyrosine kinase domain by a site directed mutagenesis to phenylalanine.

Yet still another object of the present invention is to provide a use of the protein having an increased autophosphorylation and tyrosine kinase activity as an effective target protein for developing medical drugs for treating a disease caused by an excessive autophoshorylation and tyrosine kinase activity of DDR2 protein,

To achieve these and other advantages and in accordance with the purpose of the present invention, as embodied and broadly described herein, inventors have studied the activation of autophosphorylation and tyrosine kinase activity of human DDR2 protein using an baculoviral expression system and site-directed mutagenesis to invent a method for preparing a large amount of a protein containing DDR2 cytosolic tyrosine kinase domain protein having an increased autophosphorylation and tyrosine kinase activity by achieving the phosphorylation of tyrosines at the activation loop of DDR2 cytosolic tyrosine kinase, especially by achieving the phosphorylation of at least one of tyrosines 736, 740 and 741, more especially tyrosine 740 at the activation loop of DDR2 cytosolic tyrosine kinase using a Src tyrosine kinase or Src related tyrosine kinases such as Fyn, Yes, Lck, Hck, Lyn, Csk, Blk, etc., or H O processing, or by modifying at least one of tyrosines 736, 740 and 741, more especially tyrosine 740 at the activation loop of DDR2 cytosolic tyrosine kinase by a site directed mutagenesis to other amino acid such as phenylalanine, alanine, glycine, etc.

Furthermore, the present inventors have demonstrated that the tyrosine phosphorylations plays an important role in the increase of autophosphorylation and tyrosine kinase activity towards heterologous substrates by finding that the autophosphorylation and tyrosine kinase activity towards heterologous substrates of DDR2 cytosolic tyrosine kinase domain is increased by the tyrosine phosphorylation. Furthermore, the present inventors have shown that the phosphorylation on at least one of three tyrosines (tyrosine 736, tyrosine 740 and tyrosine 741), especially tyrosine 740 in the activation loop of human DDR2 cytosolic tyrosine kinase domain is critical to activate autophosphrylation and tyrosine kinase activity towards heterologous substrates of DDR2 cytosolic domain by demonstrating that the three tyrosine residues are the target for phosphorylation by Src and the modification of tyrosine 740 to phenylalanine is enough to provide an activated autophosphorylation and tyrosine kinase activity of DDR2 cytosolic domain. This means that the invented protein containing DDR2 cytosolic tyrosine kinase domain with the enhanced autophosphorylation activity and tyrosine kinase activity towards heterologous peptide substrates by phosphorylation by Src or Src-related kinases, such as Fyn, Yes, Lck, Hck, Lyn, Csk, BIk, etc., should be characterized as having the modified tyrosine residue by phosphorylation in tyrosine 740 and/or at the same time, tyrosine 736 and/or tyrosine 741.

The DDR2 protein containing the DDR2 cytosolic tyrosine kinase domain in which tyrosines have been phosphorylated according to the present invention, is very useful in discovering a material inhibiting the autophosporylation and the tyrosine kinase activity of DDR2 cytosolic tyrosine kinase to develop a medical drug for treating the disease caused by an excessive autophosphorylation and tyrosine kinase activity of the DDR2 protein, such as liver cirrhosis, athroscrelosis, rheumatism, cancer, and the like.
First, the present invention relates to a protein containing a modified human DDR2 cytosolic tyrosine kinase domain having an increased autophosphorylation and tyrosine kinase activity, wherein tyrosine 740 in the activation loop of the human DDR2 cytosolic tyrosine kinase domain is modified by inducing a phophorylation of tyrosines 740, or by mutating to phenylalanine by a site-directed mutation. Tyrosine 740 refers to the position of the tyrosine residue in SEQ ID NO.1. The DDR2 cytosolic tyrosine kinase domain is preferable to be human DDR2 cytosolic tyrosine kinase domain.
Further, the present invention provides a method for preparing a protein containing a human DDR2 cytosolic tyrosine kinase domain having increased autophosphorylation and tyrosine kinase activity, through phosphorylation of tyrosine 740 at the DDR2 cytosolic tyrosine kinase domain, comprising the following steps of:
- amplifying DNA fragment which encodes an amino acid sequence sufficiently covering a DDR2 cytosolic tyrosine kinase domain protein, and introducing the amplified DNA fragment into a viral expression vector to construct a recombinant viral expression vector for DDR2 cytosolic tyrosine kinase domain protein and generating recombinant virus carrying the DDR2 cytosolic tyrosine kinase domain protein;
- amplifying DNA fragment which encodes an amino acid sequence sufficiently covering a full-length c-Src or c-Src related protein, and introducing the amplified DNA fragment into another separate virus expression vector genome, to construct a recombinant virus expression vector for the c-Src or c-Src related protein and generating recombinant virus carrying the c-Src or c-Src related protein;
- infecting the obtained virus carrying the DDR2 cytosolic tyrosine kinase domain and the obtained virus carrying the c-Src or c-Src related protein into a host cell, co-expressing the proteins together, and inducing a tyrosine phosphorylation at the DDR2 cytosolic tyrosine kinase domain by the tyrosine kinase activity of c-Src or c-Src related protein, to produce a large amount of a protein containing the DDR2 cytosolic tyrosine kinase domain with increased tyrosine phosphorylation;
- isolating and purifying the obtained protein containing the DDR2 cytosolic tyrosine kinase domain with increased tyrosine phosphorylation, wherein tyrosine 740 of the DDR2 cytosolic tyrosine kinase domain is essentially phosphorylated,
wherein the virus is a baculovirus and the host cell is an insect cell.
The Src related protein may be selected from the group consisting of Fyn, Yes, Lck, Hck, Lyn, Csk, Blk, etc.
Also, the present invention provides a method for preparing a protein containing a human DDR2 cytosolic tyrosine kinase domain having an increased autophosphorylation and tyrosine kinase activity, by phosphorylating tyrosine 740 at the DDR2 cytosolic tyrosine kinase domain protein, comprising the following steps of
- amplifying DNA fragment which encodes an amino acid sequence sufficiently covering a DDR2 cytosolic tyrosine kinase domain protein, and introducing the amplified DNA fragment into a viral expression vector to construct a recombinant viral expression vector for DDR2 cytosolic tyrosine kinase domain protein and generating recombinant virus carrying the DDR2 cytosolic tyrosine kinase domain protein;
- infecting the obtained the virus of the DDR2 cytosolic tyrosine kinase domain into a host cell, to produce a protein containing the DDR2 cytosolic tyrosine kinase domain, and then treating the cells with H₂O₂ at the concentration of 10 µM to 1 mM to induce tyrosine phosphorylation at the expressed DDR2 cytosolic tyrosine kinase domain; and
- isolating and purifying the expressed protein containing the DDR2 cytosolic tyrosine kinase domain with induced tyrosine phosphorylation, wherein tyrosine 740 of the DDR2 cytosolic tyrosine kinase domain is essentially phosphorylated,
wherein the virus is a baculovirus and the host cell is an insect cell.
According to the present invention, it is preferable to use human DDR2 cytosolic tyrosine kinase domain. In this case, tyrosine 740 of human DDR2 cytosolic tyrosine kinase domain is selectively phosphorylated by kinase activity of Src or Src related protein, to make it possible to increase autophosphorylation and tyrosine Idnase activity of the DDR2 cytosolic tyrosine kinase domain. The virus used in the method of the present invention is brculovirus, and the host cell is an insect cell.
Also, the present invention provides a method for preparing a protein containing a human DDR2 cytosolic tyrosine kinase domain having an increased autophosphorylation and tyrosine kinase activity, by mutating tyrosine 740 at the DDR2 cytosolic tyrosine kinase domain, comprising the following steps of:
- amplifying DNA fragment which encodes an amino acid sequence sufficiently covering a DDR2 cytosolic tyrosine kinase domain protein where tyrosine 740 at the DDR2 cytosolic tyrosine kinase domain is mutated to phenylalanine by a site-directed mutagenesis, and introducing the amplified DNA fragment into a viral expression vector to construct a recombinant viral expression vector for DDR2 cytosolic tyrosine kinase domain with mutation of tyrosine 740 to phenylalanine 740, and generating recombinant virus carrying the mutant DDR2 cytosolic tyrosine kinase domain;
- infecting the obtained recombinant virus of the mutant DDR2 cytosolic tyrosine kinase domain into a host cell, to produce a protein containing the mutant DDR2 cytosolic tyrosine kinase domain,
- isolating and purifying the expressed mutant protein containing the DDR2 cytosolic tyrosine kinase domain with mutation of tyrosine 740 to phenylalanine 740, wherein the virus is a baculovirus and the host cell is an insect cell.
In order to facilitate the purification of the expressed protein containing the DDR2 cytosolic tyrosine kinase domain or its mutant, the DNA fragment sufficiently covering the DDR2 cytosolic tyrosine kinase domain or its mutant may be tagged with an appropriate tag used for an affinity column chromatography and then introduced into the baculoviral expression vector. Glutathione-S-transferase (GST) gene, thioredoxin gene, or histidine oligomer can be used as the tag, and especially, the glutathione-S-transferase (GST) gene may be preferable.

The present invention also provides a use of a protein containing a modified DDR2 cytosolic tyrosine kinase domain having an increased autophosphorylation and tyrosine kinase activity, according to the present invention to be utilized in developing medical drugs for treating a disease caused by an excessive autophoshorylation and tyrosine kinase activity of DDR2 protein, e.g., through a screening for drug materials or a protein structure analysis of the DDR2 tyrosine kinase active domain.

DDR2 protein is a kind of a receptor protein attached to a plasma membrane. DDR2 protein is consists of three domains, i.e., an extracellular domain (i.e., N-terminal region), a transmembrane domain and a cytosolic domain (i.e., C-terminal region) exposed to cytosol. For example, a human DDR2 protein (having the amino acid sequence of SEQ ID NO: 1) consists of the extracellular domain mainly consisting of amino acids from position 1 to position 399, the transmembrane domain consisting of 22 amino aids (ILIGCLVAIIFILLAIIVIILW; SEQ ID NO: 2) following to position 399, and the intracellular cytosolic domain (C-terminal region) comprising the tyrosine kinase domain consisting of amino acids from position 441 to position 855 (SEQ ID NO: 3).

In the present invention, in order to study a change in autophosphorylation and tyrosine kinase activity of DDR2 protein by c-Src tyrosine kinase or by Src related kinases such as Fyn etc., or by processing with H ₂O₂, among a cDNA for human DDR2 protein, a cDNA fragment which encodes the amino acids from position 441 to position 855 sufficiently covering the tyrosine kinase active domain may be amplified by PCR, and introduced into a baculovirus expression vector by a conventional method. This baculovirus expression vector was used to generate a recombinant baculovirus expressing the DDR2 cytosolic tyrosine kinase domain protein. Likewise in order to study a change in autophosphorylation and tyrosine kinase activity of DDR2 cytosolic tyrosine kinase domain protein by the mutation where at least one tyrosine of three tyrosines 736, 740 and 741 are independently replaced with phenylalanine, alanine or glycine, the mutation of tyrosine 740 to phenylalanine 740 is exemplarily conducted. That is, the nucleotide sequence of TAT in the position of the codon for tyrosine 740 is replaced with the nucleotide sequence of TTT, the codon for phenylalanine in a cDNA fragment of human DDR2 which encodes the amino acids from position 441 to position 855 sufficiently covering the tyrosine kinase active domain by conventional site-directed mutagenesis method and the mutated cDNA fragment was introduced into a baculovirus expression vector by a conventional method. This constructed vector was used to generate a recombinant baculovirus expressing the mutant DDR2 cytosolic tyrosine kinase domain protein with tyrosine 740 to phenylalanine 740 mutation.

Preferably, in order to facilitate the purification of the expressed DDR2 protein, the cDNA fragment comprising the DDR2 cytosolic tyrosine kinase domain protein and its mutant may be tagged with glutathione-S-transferase (GST) gene, thioredoxin gene, histidine oligomer or the like, which can be used in an affinity tagging, and then introduced into a virus.

In a preferred embodiment of the present invention, the cDNA fragment encoding the DDR2 cytosolic tyrosine kinase domain protein or its mutant protein may be attached to the C-terminal coding region (3' region) of the glutathione-S-transferase gene, and the fused DNA fragment is cloned into a baculovirus expression vector pBacPAK8 (Clontech, USA) by using a conventional gene recombination technique, to construct a recombinant bxulovirus capable to produce a fused protein of the glutathione-S-transferase and the DDR2 cytosolic tyrosine kinase domain protein or its mutant protein (CHLONTECH BacPAKTM Baculovirus Expression System User Manual, PT1260-1 (PR95847), Published 12 May, 1999. Catalog # K1601-1).

The DDR2 protein to be used is not limited to human DDR2 protein, and the cDNA may be not limited in the number of amino acids encoded thereby so long as it contains the sequence encoding the tyrosine kinase active domain of DDR2 protein. The nucleotide sequence used for mutations of tyrosine 736 and/or tyrosine 740 and/or tyrosine 741 is not limited to TTT corresponding to phenylalanine thereby so long as it contains the sequence coding for phenylalanine or other appropriate amino acid such as alanine or glycine. Since tagging the DDR2 gene fragment with a tagging materials, such as Glutathione-S-transferase (GST) gene, is only for facilitating the purification of the expressed DDR2 protein, such tagging process is not always essential for the present invention. The DDR2 gene fragment may be used in a fused form, or it may be also used after removing the GST portion by using an appropriate protease, according to its use after completing of purification process.

In the present invention, in order to induce the tyrosine phosphorylation at the DDR2 cytosolic tyrosine kinase domain, a gene (SEQ ID NO: 4) encoding a full-length c-Src tyrosine kinase or a gene (SEQ ID NO: 5) encoding a full-length c-Fyn tyrosine kinase may be amplified by PCR, and then the PCR amplified gene may be cloned into a baculovirus expression vector, to generate a recombinant baculovirus expressing the full-length c-Src tyrosine kinase or the full-length c-Fyn tyrosine kinase.

In an embodiment of the present invention, in order to induce the tyrosine phosphorylation at the DDR2 kinase active domain, a full-length c-Src protein or a full-length c-Fyn protein may be co-expressed in insect cells used as a host cell. However, a c-Src protein fragment having a sufficient c-Src kinase activity, or a kinase having a peptide substrate specificity equal to v-Src or c-Src, or a c-Fyn protein fragment having a sufficient c-Fyn kinase activity, or a kinase having a peptide substrate specificity equal to v-Fyn or c-Fyn may also be used instead of the full-length c-Src protein or the full-length c-Fyn protein to induce the tyrosine phosphorylation at the DDR2 cytosolic tyrosine kinase domain.

In another embodiment of the present invention, insect cell, sf9 or sf21 or High-five cell which is conventionally used for protein expression by the baculovirus, may be used as a host cell.

Alternatively, in the present invention, the tyrosine phosphorylation at the DDR2 cytosolic tyrosine kinase domain may be induced by processing the host cells expressing DDR2 cytosolic tyrosine kinase domain with H₂O₂ . In a preferred embodiment of such tyrosine phosphorylation induction at the DDR2 cytosolic tyrosine kinase domain by H₂O₂ processing, the protein in which the tyrosine phosphorylation is induced at the **DDR2** cytosolic tyrosine kinase domain may be obtained by infecting the recombinant baculovirus, in which a DNA fragment encoding **DDR2** cytosolic tyrosine kinase domain is introduced, at the MOI (multiplicity of infection) of 1 to 10, into an appropriate insect cells, according to the aforementioned method; after 24 to 72 hours, directly adding H O at the concentration of 10 µM to 1 mM to the incubated cells and maintaining for 15 minutes to 1 hour; collecting the cells; and isolating and purifying a protein containing the DDR2 cytosolic tyrosine kinase domain.

The obtained tyrosine phosphorylation-induced DDR2 cytosolic tyrosine kinase domain fused with the Glutathione-S-transferase may be purified in a high purity by an affinity chromatography according to a conventional method using glutathione-attached beads.

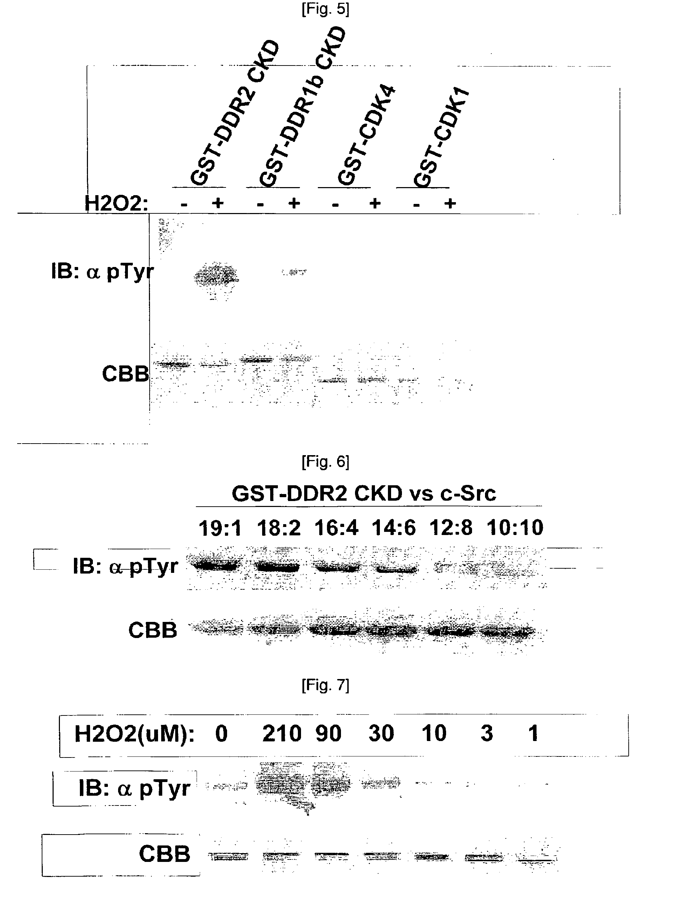

In the preferred embodiment of the present invention, the intracellular co-expression of the fused protein of the glutathione-S-transferase and the DDR2 cytosolic tyrosine kinase domain, and the c-Src tyrosine kinase, in insect cells, may be achieved by simultaneously infecting two recombinant baculoviruses into insect cell sf9, at the proper combination ratio of 1:3 to 3:1 and the MOI of 1 to 10, and then, maintaining for 24 to 72 hours. The generated baculoviral titers that are normally more than 10⁸ pfu /ml can be preferably used. The present inventors have found that as long as the ratio of baculoviral titer of c-Src versus that of DDR2 cytosolic tyrosine kinase domain was more than 2:3 in this co-infection into sf9 cells with MOI of 10, the degree of tyrosine phosphorylation in a specific amount of co-expressed DDR2 cytosolic tyrosine kinase domain protein remained almost constant even though the ratio was increased. Therefore, as a preferable method, a saturated tyrosine phosphorylation of DDR2 cytosolic tyrosine kinase domain by c-Src can be easily achieved by coinfecting the both viral titers in a ratio of 1:1 with MOI of 10 into sf9 cells. Increase of this ratio resulted in the decrease the recovery of the expressed DDR2 cytosolic tyrosine kinase domain protein in insect cells as shown in figure 6.

In the preferred embodiment of the present invention, the intraxellular co-expression of the fused protein of the glutathione-S-transferase and the DDR2 cytosolic tyrosine kinase domain, and the c-Fyn tyrosine kinase, in insect cells, may be achieved by simultaneously infecting two recombinant baculoviruses into insect cell sf9, at the proper combination ratio of 1:3 to 3:1 and the MOI of 1 to 10, and then, maintaining for 24 to 72 hours. The generated baculoviral titers that are normally more than 10⁸ pfu /ml can be preferably used. Likewise to the intraxellular co-expression of the fused protein of the glutathione-S-transferase and the DDR2 cytosolic tyrosine kinase domain, and the c-Fyn tyrosine kinase, in insect cells , as a preferable method, a saturated tyrosine phosphorylation of DDR2 cytosolic tyrosine kinase domain by c-Fyn with a high yield can be easily achieved by coinfecting the both viral titers in a ratio of 1:1 with MOI of 10 into sf9 cells.

As mentioned above, the DDR2 cytosolic tyrosine kinase domain fused the glutathione-S-transferase after co-expressing with the Src protein or the c-Fyn protein in insect cells may be purified in a high purity, by lysing the cells, and carrying out a glutathione agarose affinity column chromatography according to a conventional method. Wherein, it can be proved that the Src kinase protein or Fyn protein is not contained in the purified DDR2 cytosolic tyrosine kinase domain fused to glutathione-S-transferase, through a western blotting test using an Src or c-Fyn specific antibody.

The present inventors carried the experiment to elucidate the site(s) of tyrosine phosphorylation in DDR2 cytosolic tyrosine kinase domain by c-Src. For this, the baculoviral expression vector of kinase defective DDR2 cytosolic tyrosine kinase domain was generated by the site-directed mutagenesis where lysine 608 of human DDR2 is mutated to alanine 608. The reason for the kinase defective DDR2 cytosolic tyrosine kinase mutant being used was to abolish the tyrosine phosphorylation effect by DDR2 autophosphorylation activity so that the observed tyrosine phosphorylation in DDR2 kinase defective cytosolic domain came only from Src tyrosine kinase activity.

The generated baculoviral expression vector of kinase defective DDR2 cytosolic tyrosine kinase domain was further subjected to the site-directed mutagenesis to replace the three tyrosines in the activation loop of DDR2 tyrosine kinase with phenylalanine(s) in single or by pairs or in all three tyrosine residues.

The baculoviral expression vector of kinase defective DDR2 cytosolic tyrosine kinase domain and its five mutants carrying mutation in the three tyrosine residues such as Y736F (means the mutation where tyrosine 736 is mutated to phenylalanine), Y740F (means the mutation where tyrosine 740 is mutated to phenylalanine), Y736/741F (means the mutation where both of tyrosine 736 and tyrosine 741 are mutated to phenylalanines), Y740/741F (means the mutation where both of tyrosine 740 and tyrosine 741 are mutated to phenylalanines), and Y736/740/741F (means the mutation where all three tyrosines of 736, 740 and 741 are mutated to phenylalanines) are used to generate the corresponding baculovirus.

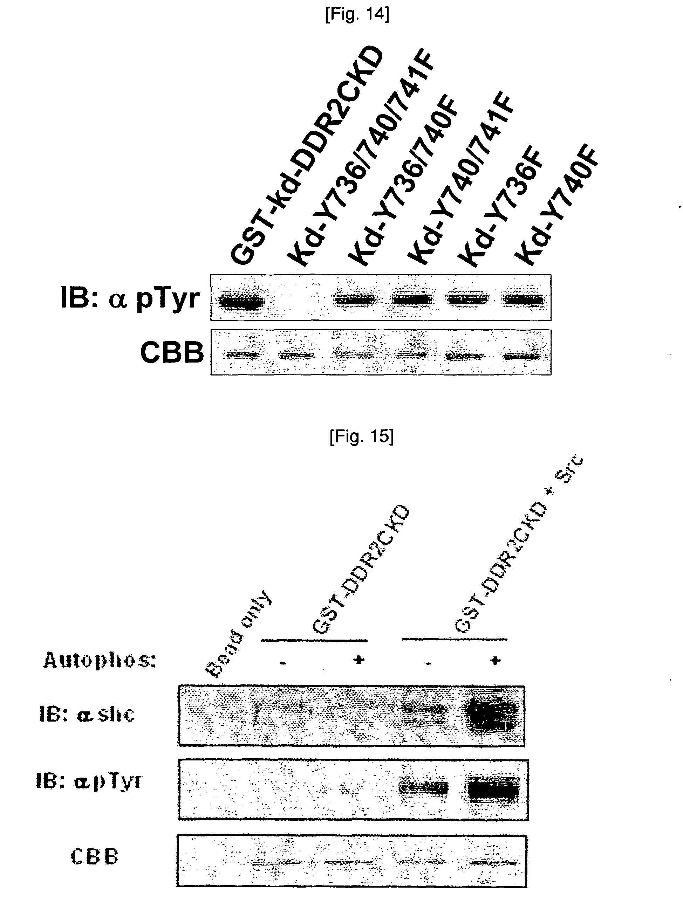

The each of six baculoviruses described in above was co-infected into sf 9 cells with the baculovirus carrying c-Src to express two proteins simultaneously in sf9 cells, and the expressed kinase defective DDR2 cytosolic tyrosine kinase domain as well as its five mutants was purified using glutathione bead and examined whether the mutation in three tyrosine residues could change the degree of tyrosine phosphorylation by Src by western blotting on the glutathione bead purified proteins using an phospho-tyrosine specific antibody. The tyrosine phosphorylation was disappeared in the mutant of the all three tyrosine residues mutated to phenylalanines. In case of mutants such as Y736F, Y740F, Y736/741F, and Y740/741F, they show still a tyrosine phosphorylation, but with a slightly reduced level, compared with the kinase defective GST-DDR2 cytosolic domain with all three tyrosine residues unchanged as shown in figure 14. These results indicate that tyrosine phosphorylation by Src happens within the three tyrosine residues in the activation loop of DDR2.

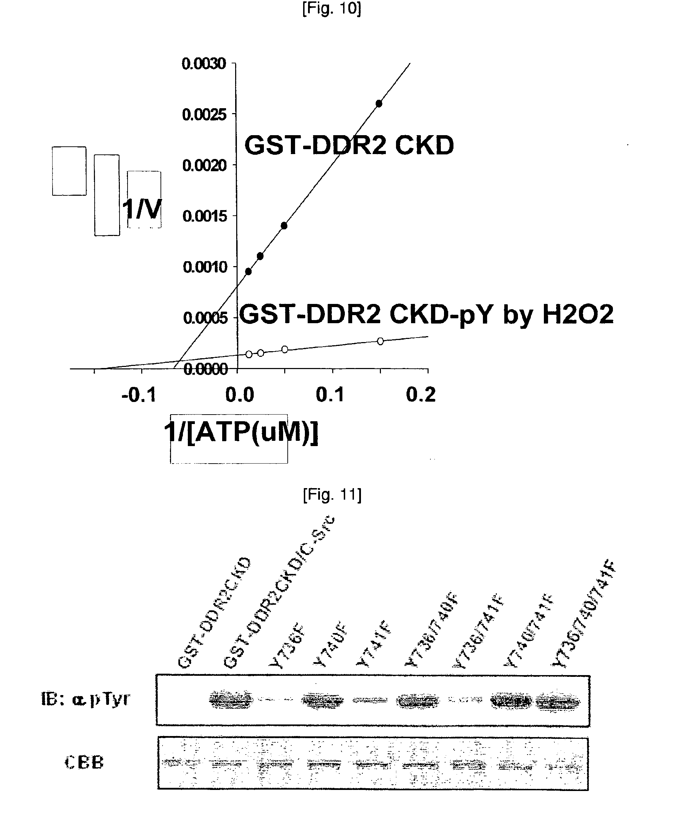

The present inventors carried further experiments to elucidate the role of three tyrosine residues in the activation of autophosphorylation and tyrosine kinase activity of DDR2 cytosolic tyrosine kinase domain. From this experiment the present inventors invented that the mutant of human DDR2 cytosolic tyrosine kinase domain where tyrosine 740 is replaced with phenylalanine 740 has the character of activated autophosphorylation and tyrosine kinase activity as same as the DDR2 cytosolic tyrosine kinase domain phosphorylated by c-Src or c-Fyn as shown in figure 11, 12 and 13. This result indicate that tyrosine 740 plays a inhibitory role against the autophosphorylation and tyrosine kinase activity of DDR2 cytosolic tyrosine kinase domain and the modification of this tyrosine 740 by mutation to a different amino acid like phenylalanine or by phosphorylation with the means of c-Src or c-Fyn prevent tyrosine 740 from inhibiting the autophosphorylation and tyrosine kinase activity of DDR2 cytosolic tyrosine kinase domain. Therefore this result further indicates that the critical tyrosine residue to be phosphorylated by c-Src or c-Fyn in the DDR2 cytosolic tyrosine kinase domain with the activated autophosphorylation and tyrosine kinase activity is the tyrosine 740.

In the preferred embodiment of the present invention, the nucleotide sequence of TAT in the position of the codon for tyrosine 740 is replaced with the nucleotide sequence of TTT, a codon for phenylalanine in a cDNA fragment of human DDR2 which encodes the amino acids from position 441 to position 855 sufficiently covering the tyrosine kinase active domain by conventional site-directed mutagenesis method. The mutated cDNA fragment may be attached to the C-terminal coding region (3' region) of the glutathione-S-transferase gene and introduced into a baculovirus expression vector pBacPAK8 (Clontech, USA) by a conventional method. In this invention, other codon sequences of phenylalanine or the codon sequence of an appropriate amino acid instead of phenylalanine can be used. This constructed vector was used to generate a recombinant baculovirus expressing the mutant DDR2 cytosolic tyrosine kinase domain protein with tyrosine 740 to phenylalanine 740 mutation (CHLONTECH BacPAKTM Baculovirus Expression System User Manual, PT1260-1 (PR95847), Published 12 May, 1999. Catalog #K1601-1). The generated baculoviral titers that are normally more than 10⁸ pfu /ml can be preferably used. The baculovirus is infected to insect cells such as sf9 with MOI 10 and maintained for 24-72 hours and cells are lysed after harvest and the expressed fusion protein of GST and DDR2 cytosolic tyrosine kinase domain protein with the mutation at tyrosine 740 to phenylalanine 740 is purified using glutathione bead by a conventional affinity column chromatography.

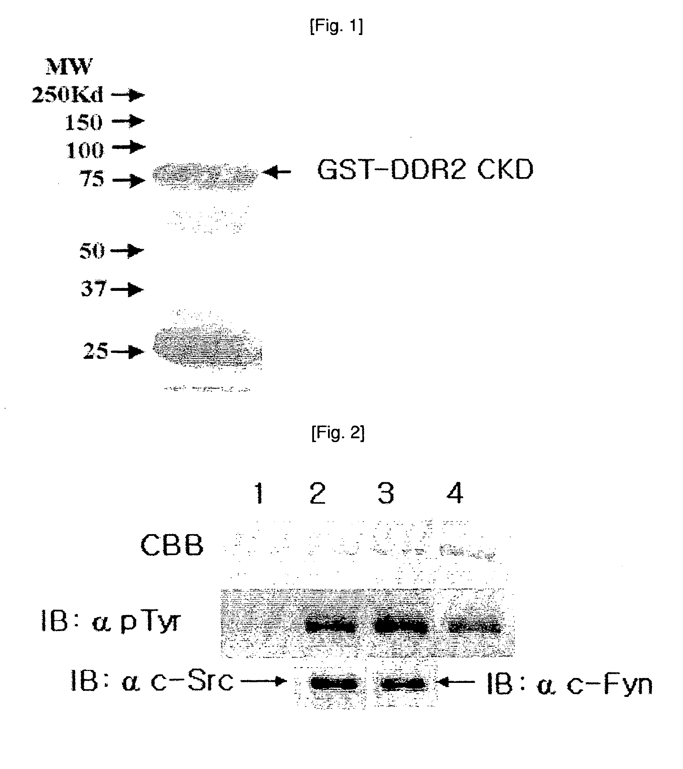

Figure 1 shows a representative result obtained by the fusion protein of glutathione S transferase and human DDR2 cytosolic tyrosine kinase domain (GST-DDR2 CKD) through the expression in insect cells, and a glutathione agarose bead column chromatography, performing electrophoresis in 10 % polyacrylamide gel, and then staining with coomassie. As shown in Figure 1, the purified protein has the molecular weight of 75,000 Da, which is an expected molecular weight of the GST-DDR2 CKD. It has been checked that the protein having the molecular weight of 75,000 Da corresponds to the DDR2 cytosolic tyrosine kinase domain fused to glutathione S transferase, by the western blotting test using a specific antibody against the C-terminus of human DDR2 protein.

As an interesting result in the present invention, as shown in figure 2, it has been checked, by the western blotting test using a phosphorylated tyrosine specific antibody, that when the fusion protein (GST-DDR2 CKD) of the glutathione-S-transferase and the DDR2 cytosolic tyrosine kinase domain is co-expressed together with the c-Src tyrosine kinase, or with the c-Fyn tyrosine kinase in insect cells or the GST-DDR2 CKD is solely expressed in insect cells and then processed with H O of 200 uM for half of an hour, the tyrosine phosphorylation is remarkably induced in the fused protein of GST-DDR2 CKD.

Figure 2 shows the results obtained by purifying the GST-DDR2 CKD from the expressed insect cells using glutathione agarose beads, and performing poly-acrylamaide gel electrophoresis (PAGE) followed by the western blotting test using a phosphorylated tyrosine (p-tyrosine) specific antibody wherein the samples are obtained by solely expressing the fused protein GST-DDR2 CKD in insect cells (lane 1), co-expressing the GST-DDR2 CKD together with the c-Src protein in insect cells (lane 2), and co-expressing the GST-DDR2 CKD together with the c-Fyn protein in insect cells (lane 3), solely expressing the fused protein GST-DDR2 CKD and then processing the expressed GST-DDR2 CKD in insect cells with H₂O₂ at the concentration of 200 uM for half of an hour (lane 4). The presence of the purified GST-DDR2 CKD having the molecular weight of 75,000 Da was proved by coomassie staining. In order to check the expression of c-Src protein in the case of co-expressing the GST-DDR2 CKD and the c-Src (lane 2), a portion of lysate of the host cells is undergone a SDS-PAGE and then a western blotting using a c-Src protein specific antibody. Likewise in order to check the expression of c-Fyn protein in the case of co-expressing the GST-DDR2 CKD and the c-Fyn (lane 3), a portion of lysate of the host cells is undergone a SDS-PAGE and then a western blotting using a c-Fyn protein specific antibody.

As can be seen from Figure 2, it can be proved that the tyrosine phosphorylation is induced at the DDR2 cytosolic tyrosine kinase domain by the H₂O₂ processing or the co-expression with the Src protein or the co-expression with the Fyn protein.

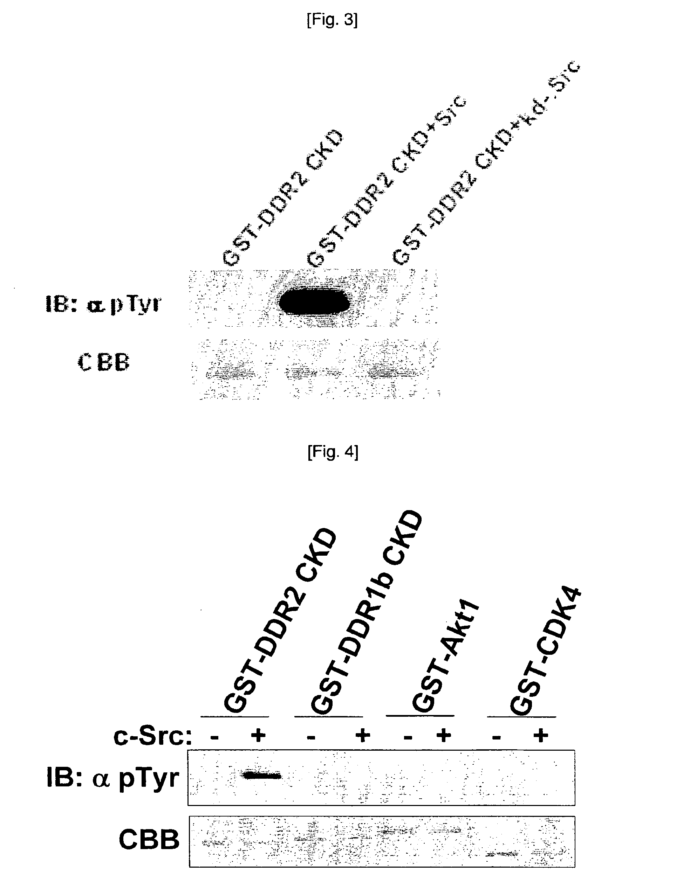

Through the western blotting test using a c-Src protein specific antibody, it has been checked that the c-Src protein coding recombinant baculovirus expresses the c-Src protein in insect cells, and the result has been also shown in Figure 2. Likewise, through the western blotting test using a c-Fyn protein specific antibody, it has been checked that the c-Fyn protein coding recombinant baculovirus expresses the c-Fyn protein in insect cells, and the result has been also shown in Figure 2. In an embodiment of the present invention, the human c-Src gene or the human c-Fyn gene may be used to introduce the tyrosine phosphorylation at the DDR2 cytosolic tyrosine kinase domain. Alternatively, the same results can be obtained by using anc- Src gene of other species, a modified v-Src gene or an c-Fyn gene of other species, a modified v-Fyn gene or a gene for a kinase having peptide substrate specificity similar to the Src or the Fyn protein. However, as shown in Figure 3 in the same test using a mutated Src protein which has no kinase activity (kd-Src), it is shown that the tyrosine phosphorylation at the cytosolic tyrosin kinase domain has not been induced, whereby it can be proved that the Src kinase activity is associated with the tyrosine phosphorylation at the DDR2 cytosolic tyrosine kinase domain. From a similar experiment, it is shown that Fyn tyrosine kinase activity is required to induce the tyrosine phosphorylation in DDR2 cytosolic tyrosine kinase domain.

figure 4 shows that the induction of the tyrosine phosphorylation by the co-expression with the Src protein is specific to the tyrosine kinase active domain of the DDR2 protein. The results shown in Figure 4 were obtained by co-expressing a fused protein GST-DDR2 CKD, a fused protein (GST-DDR1b CKD) of the GST and the human DDR1b cytosolic tyrosine kinase domain, a fused protein (GST-Akt1) of the GST and the full-length mouse Akt1 kinase and a fused protein (GST-CDK4) of the GST and the full-length human CDK4 kinase with the c-Src protein without GST tagging in insect cells, respectively; purifying the respective expressed product using glutathione beads; performing SDS-PAGE; and performing the western blotting test using a phosphorylated tyrosine specific antibody and ooomassie staining. From the results of coomassie staining, the presence of the respective fused protein can be proved. From the results of the western blotting test using a phosphorylated tyrosine specific antibody, It can be seen that the tyrosine phosphorylation is strongly induced in the GST-DDR2 CKD, while the tyrosine phosphorylation is poorly induced or never induced in the GST-DDR1b CKD, the GST-Akt1 and the GST-CDK4 used as control groups. Therefore, it can be proved that the tyrosine phosphorylation induced by the co-expression with the c-Src tyrosine kinase is specific to the DDR2 cytosolic tyrosine kinase domain.

Figure 5 shows that the tyrosine phosphorylation by H₂O₂ processing is specific to the DDR2 cytosolic tyrosine kinase domain. The results shown in Figure 5 were obtained by solely expressing the fused proteins, GST-DDR2 CKD, the GST-DDR1b CKD, the GST-CDK4 and the GST-CDK1, respectively, in insect cells; processing with 200 µM of H₂O₂ for half of an hour; purifying the respective expressed product using glutathione beads; performing the SDS-PAGE; and performing the western blotting test using an phosphorylated tyrosine specific antibody antibody and coomassie staining. From the results of coomassie staining, the presence of the respective fused protein can be proved. From the results of the western blotting test using a phosphorylated tyrosine specific antibody, It can be seen that the tyrosine phosphorylation is strongly induced in the GST-DDR2 CKD, while the tyrosine phosphorylation is poorly induced or never induced in the GST-DDR1b CKD, the GST-CDK4 and the GST-CDK1 used as control groups. Therefore, it can be proved that the tyrosine phosphorylation induced by processing with H₂O₂ is also specific to the DDR2 cytosolic tyrosine kinase domain.

Figure 6 shows the results showing a variation of an amount of the expressed GST-DDR2 CKD and the level of the tyrosine phosphorylation, depending on the ratio between two baculoviruses of which one expresses the DDR2 cytosolic tyrosine kinase domain and the other expresses the c-Src protein, when the two baculoviruses are simultaneously infected to the host cell with the total MOI of 10. The results have been obtained by co-infecting the GST-DDR2 CKD expression baculovirus and the Src expression baculovirus in insect cells by the titer ratio of 19:1, 18:2, 16:4, 14:6, 12:8 and 10:10, respectively; purifying the GST-DDR2 CKD using glutathione beads; performing the SDS-PAGE; and quantifying of the purified GST-DDR2 CKD by the coomassie staining and performing the western blotting of the protein developed in SDS-PAGE using a phosphorylated tyrosine specific antibody.

As can be seen from Figure 6, as the higher ratio of the Src expression virus versus GST-DDR2 CKD virus is, the higher level of the tyrosine phosphorylation at a unit amount of the DDR2 cytosolic tyrosine kinase domain protein is obtained. This means that the induction of the tyrosine phosphorylation at the DDR2 cytosolic tyrosine kinase domain by the c-Src protein depends on the concentration of the c-Src protein. The tyrosine phosphorylation at the DDR2 cytosolic tyrosine kinase domain was meaningfully induced, when the DDR2 kinase expression virus and the c-Src protein expression virus are co-expressed even at the ratio of 19:1. However Figure 6 shows that in order to obtain the saturated level of tyrosine phosphorylation in DDR2 cytosolic tyrosine kinase domain, the ratio of the titer of the baculovirus coding for c-Src to the titer of baculovirus coding for DDR2 cytosolic tyrosine kinase domain is necessary more than 2 to 3. Increase of this ratio causes the reduction in the yield of expressed DDR2 cytosolic tyrosine kinase domain. Therefore it is appropriate to infect the baculovirus of src and the baculovirus of DDR2 cytosolic tyrosine kinase domain by a ratio of 1:1 to the insect cells to obtain DDR2 cytosolic tyrosine kinase domain protein with the saturated level of tyrosine phosphorylation as well as the high yield.

Figure 7 shows the results showing a variation of an expressed amount of the GST-DDR2 CKD and the level of tyrosine phosphorylation depending on concentration of H₂O₂ to be processed. The results have been obtained by solely expressing the GST-DDR2 CKD in insect cells; processing the cell with H₂O₂ at the concentration of 0, 1, 3, 10, 30, 90 and 210 µM, respectively, for half of an hour; purifying the obtained GST-DDR2 CKD protein using glutathione beads; and determining the amount of the purified DDR2 cytosolic tyrosine kinase domain and the level of the tyrosine phosphorylation by performing coomassie staining and the western blotting using a phosphorylated tyrosine specific antibody. As can be seen from Figure 7, it can be seen that the level of the tyrosine phosphorylation at the DDR2 cytosolic tyrosine kinase domain by the H₂O₂ processing depends on the concentration of H₂O₂.

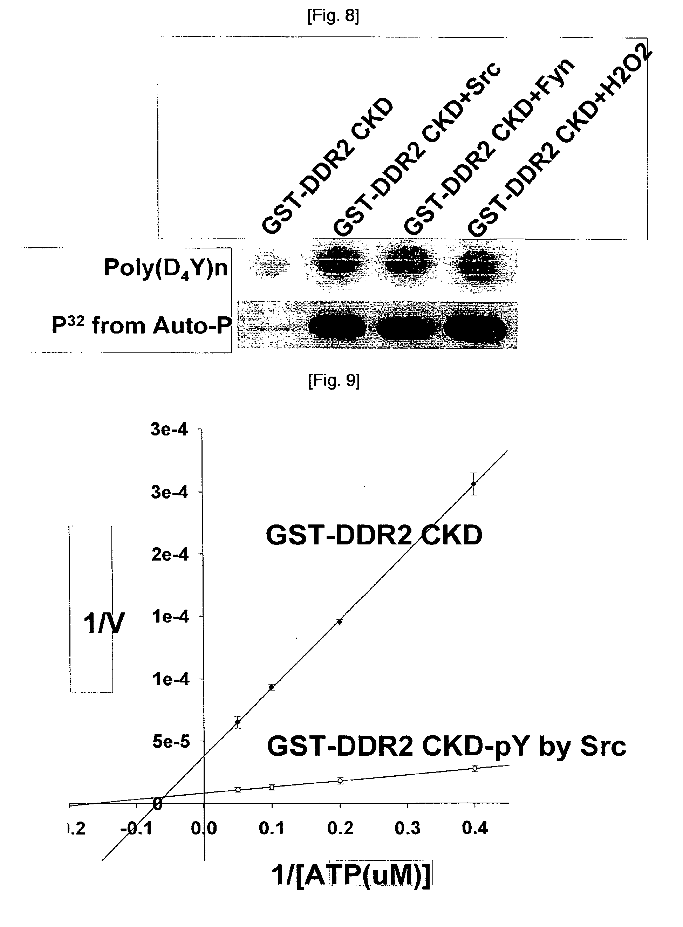

In order to show the functional difference between the DDR2 tyrosine kinase protein in which tyrosine at the tyrosine kinase domain has been phosphorlated by the co-expression with the Src tyrosine kinase or by the co-expression with the Fyn tyrosine kinase or the H₂O₂ processing according to the present invention, and another DDR2 tyrosine kinase (control protein) in which no tyrosine phosphorylation has been induced, the enzymatic activities of both the tyrosine-phosphorylated DDR2 tyrosine kinase and the non-phosphorylated DDR2 tyrosine kinase have been measured and compared respectively, and the results are shown in Figure 8. The results in Figure 8 have been obtained by measuring and comparing the tyrosine kinase activities by the typical methods (Refer to Promega, 2001, Catalog #15.18) using a biotin-attached poly(D₄Y)n substrate (Promega, U.S.A) which is conventionally used in a method for measuring a tyrosine kinase enzyme activity. The results show the DDR2 cytosolic tyrosine kinase domain in which tyrosine has been phosphorylated by the three methods of the present invention, i.e., the co-expression with the c-Src protein or the co-expression with the c-Fyn protein or the H₂O₂ processing, has increased tyrosine kinase activity by about 5 to 10 times comparing to the control protein in which the tyrosine phosphorylation has not been induced. In measuring auto-phosphorylation activity it has been shown that the auto-phosphorylation activity of the tyrosine phosphorylated DDR2 cytosolic tyrosine kinase domain of the present invention is increased by about 5 to 10 times as well.

As a measure for searching the reason of such increase of the kinase activity, Figure 9 shows the results obtained by determining the variation of a reaction depending on the concentration of ATP, which is one of substrates for the kinase enzyme, and then conducting a reciprocal plotting (Lubert Stryer, Biochemistry Fourth Edition, Published in Seoul Foreign Books, pp. 202-205). The GST-DDR2 CKD was solely expressed or co-expressed with the c-Src protein at the ratio of 1:1 in insect cells; the respective obtained GST-DDR2 CKDs were purified; and the tyrosine kinase activities depending on the concentration of ATP were determined using the same amounts of the tyrosine phosphorylation-induced GST-DDR2 CKD (p-GST-DDR2 CKD by Src) which is obtained by co-expression with the c-Src protein in sf 9 cells and non-phosphorylated GST-DDR2 CKD which is obtained by solely expression in sf9 cells. As seen from figure 9, with comparison to that of the non-phosphorylated control protein, Km value (a reciprocal of x-axis intercept) of the tyrosine-phosphorylated DDR2 kinase protein for ATP has been decreased, while Vmax (a reciprocal of y-axis intercept) has been increased.

Figure 10 shows the results obtained by expressing GST-DDR2 CKD in insect cells; processing with 200 nM H₂O₂ for 30 minutes; purifying the obtained GST-DDR2 CKD; determining the tyrosine kinase activities depending on the concentration of ATP using the same amounts of the tyrosine phosphorylation-induced GST-DDR2 CKD (p-GST-DDR2 CKD by H₂O₂) by H₂O₂ processing and non-phosphorylated GST-DDR2 CKD which is not subject to H ₂O₂ processing; and conducting a reciprocal plotting. As can be seen from Figure 10, with comparison to that of the non-phosphorylated control protein, Km value (a reciprocal of x-axis intercept) of the tyrosine-phosphorylated DDR2 kinase protein for ATP has been decreased, while Vmax (a reciprocal of y-axis intercept) has been increased. Those results show that for the tyrosine-phosphorylated DDR2 tyrosine kinase protein, a binding capacity to the substrate has been increased and the efficiency of enzymatic reaction has been also increased, by the tyrosine phosphorylation. These facts suggest that in DDR2 kinase, an enzymatic active pocket region containing an ATP-binding site, which is generally considered as a main target point in developing a kinase inhibitor, may be structurally changed due to the tyrosine phosphorylation.

Figure 11 shows that the phenylalanine mutation of tyrosine 740 in human DDR2 cytosolic tyrosine kinase domain is enough to induce the tyrosine phosphorylaton when it is expressed in sf 9 insect cells. Various phenylalanine mutations such as Y736F (the mutation of tyrosine 736 to phenylalanine 736) , Y740F (the mutation of tyrosine 740 to phenylalanine 740), Y741 ((the mutation of tyrosine 741 to phenylalanine 741), Y736/740F (the mutation of both of tyrosines 736 and 740 to phenylalanines of 736 and 740), Y736/741F (the mutation of both of tyrosines 736 and 741 to phenylalanines of 736 and 741), Y740/741F (the mutation of both of tyrosines 740 and 741 to phenylalanines of 740 and 741), Y736/740/741F (the mutation of all three tyrosines of 736,740 and 741 to phenylalanines) in the three tyrosine residues of the activation loop of **DDR2** cytosolic tyrosine kinase domain were generated by the site directed mutagenesis methods and each wild or **DDR2** cytosolic tyrosine kinase domain mutant was infected to sf 9 cells and harvested after 2 days, then purified using glutathione bead. Each equal amount of the purified protein as estimated by coomassie staining was tested for the degree of tyrosine phosphorylation using phospho-tyrosine specific antibody. As appeared in figure 11, any mutant as long as it has the mutation in tyrosine 740 to phenylalanine, shows an enhanced tyrosine phosphorylaton as much as observed in the DDR2 cytosolic tyrosine kinas domain that is co-expressed with c-Src in sf 9 cells by co-infecting both baculoviruses in a ratio of 1 to 1.

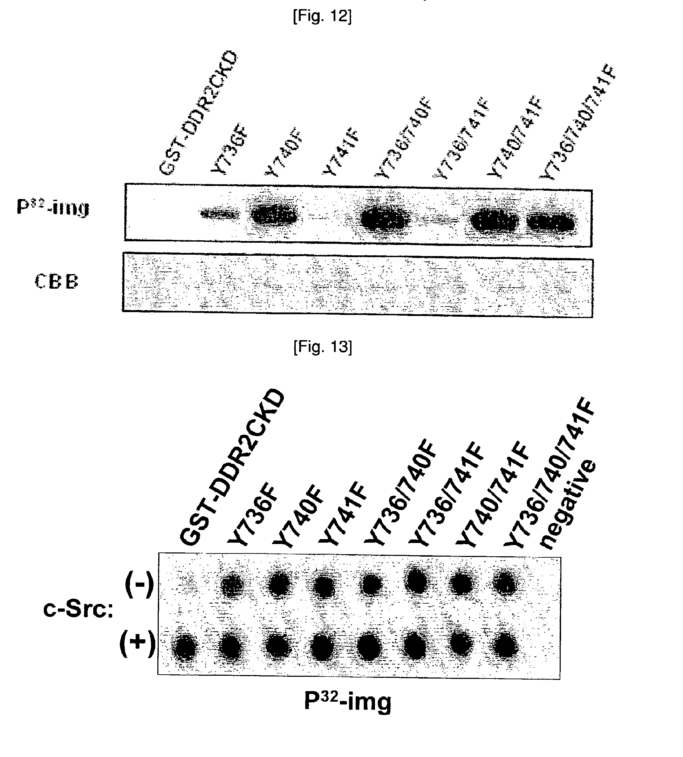

Figure 12 shows that any mutant as long as it has the mutation in tyrosine 740 to phenylalanine, shows an enhanced autophosphorylation activity when the autophosphorylation activity of each purified protein is measured and its enhanced autophosphorylation activity is almost as much same as observed in the DDR2 cytosolic tyrosine kinas domain that is co-expressed with c-Src in sf 9 cells by co-infecting both baculoviruses in a ratio of 1 to 1. This result indicates the modification of tyrosine 740 to phenylalanine is enough to activate the autophosphorylation activity of DDR2 cytosolic tyrosine kinase domain.

Figure 13 shows that all the mutants described above have enhanced tyrosine kinase activity as much as observed in the DDR2 cytosolic tyrosine kinase domain that is co-expressed with c-Src in sf 9 cells by co-infecting both baculoviruses in a ratio of 1 to 1 when their tyrosine kinase activities are measured toward Histone H2B and this enhanced activity is not significantly increased even when the mutant proteins which are purified after co-expression with c-Src in sf 9 cells by co-infecting both baculoviruses in a ratio of 1 to 1 are used. This result indicates that the tyrosine 740 mutation to phenylalanine as well as other mutations in tyrosines of 736 and/or tyrosine 741 is enough to activate the tyrosine kinase activity of DDR2 cytosolic tyrosine kinase domain as much as observed in the DDR2 cytosolic tyrosine kinas domain that is co-expressed with c-Src in sf 9 cells by co-infecting both baculoviruses in a ratio of 1 to 1.

Having seen that 740 mutation on the increase of DDR2 tyrosine kinase activity as well as its autophosphorylation activity as Src or Fyn did, The present inventors examined that the regulation of DDR2 tyrosine kinase activity and its autophosphorylation by Src or Fyn might be mediated through the phosphorylation of tyrosine 740. In addition It is probable that the all three tyrosine residues could be the targets for phosphorylation by Src kinase activity because all tyrosine kinases are equally involved in preventing the activation of DDR2 tyrosine kinase even though Tyr740 is the only critical residues regulating both of its kinase activity and autophosphorylation. To address whether the three tyrosines in the activation loop of DDR2 can be phosphorylated by Src, we generated seven single or combinational phenylalanine mutations in the three tyrosine residues of DDR2 tyrosine kinase activation loop in the background of the kinase defective DDR2 cytosolic tyrosine kinase domain where lysine 608 is mutated to alanine 608 (GST-kd-DDR2 CKD (K608A)). The reason we used the kinase defective DDR2 cytosolic tyrosine kinase mutant was to abolish the tyrosine phosphorylation effect by DDR2 autophosphorylation activity so that the observed tyrosine phosphorylation in DDR2 kinase defective cytosolic domain came only from Src tyrosine kinase activity. When the present inventors expressed these mutants in sf9 cells using baculoviral expression, we could get a stably soluble expression only from the kinase defective DDR2 cytosolic domain mutants having the mutations of Y736F, Y740F, Y736/741F, Y740/741F, and Y736/740/741F among the seven mutants and other two mutants failed to give a stable expression probably due to an abnormal change of the protein conformation. The five mutant proteins to give stable expressions were coexpressed with Src in sf9 cells and examined whether the mutation in three tyrosine residues could change the degree of tyrosine phosphorylation by Src. As shown in figure 14, in western blotting on the glutathione bead purified proteins using an phosphotyrosine specific antibody, the tyrosine phosphorylation is disappeared in the mutant of the all three tyrosine residues mutated to phenylalanines. In case of mutants such as Y736F, Y740F, Y736/741F, and Y740/741F, they show still a tyrosine phosphorylation, but with a slightly reduced level, compared with the kinase defective GST-DDR2 cytosolic domain with all three tyrosine residues unchanged. This result demonstrates that tyrosine phosphorylation by Src happens within the three tyrosine residues in the activation loop of DDR2 and tyrosine 740 can be the target for phosphorylation by Src. In addition to tyrosine 740, other two tyrosines also seem to be subject to phosphorylation by Src. Taken together with the results shown in figures 11, 12, and 13, this result can be interpreted that the phosphorylation of tyrosine 740 is a key event for DDR2 tyrosine kinase activation by Src and in case that two tyrosine residues besides tyrosine 740 are phosphorylated first by Src, they would assist tyrosine 740 to be phosphorylated.

One of the characteristic features of tyrosine phosphorylated cytosolic domain of receptor tyrosine kinase is to recruit the signaling proteins such as she protein. This complex formation between the cytosolic domain of receptor tyrosine kinase protein and signaling proteins such as she is important for the cellular transmission of the activated receptor tyrosine kinase signal. It has been observed in HSC T6 liver stellate cells and fibroblast cells that shc, one of signaling protein containing PTB domain binds to activated DDR2 cytosolic domain. The present inventors examined whether the invented DDR2 cytosolic tyrosine kinase domain protein with induced tyrosine phosphorylation for example, by the method of co-expression with c-Src in sf 9 cells or with the mutation of tyrosine 740 to phenylalanine 740 can be used for in vitro shc binding assay. In figure 15, the purified GST-DDR2 cytosolic domain from sf 9 cells with and without Src coinfection were used for in vitro she binding assay. Each protein was subject to autophosphorylation reaction at 30°C for 15 min, and a mock-autophosphylation reaction as well where gamma-S-ATP was used instead of ATP in the reaction buffer before they were used for in vitro she binding assay using HSC T6 cell lysate. DDR2 c-terminal protein did not attract she protein binding significantly. The in vitro autophosphrylation reaction of this protein did not give significant tyrosine phosphorylation as expected and this was correlated with a failure to show an improved shc binding. On the contrary, DDR2 pre-phosphorylated by co-expression of Src in sf9 cells showed some shc binding and this binding was significantly increased by in vitro autophosphorylation reaction that gave an additionally enhanced tyrosine phosphorylation. This result demonstarte that autophosphorylation of DDR2 cytosolic domain is necessary for shc binding to it and some phospho-tyrosine residue(s) resulted from autophosphorylation is critically involved for shc binding.

Next the present inventors performed the shc binding assay with the purified mutant proteins of Y740F and Y736/740/741F. As shown in figure 11 and 7b, mutants of Y740F and Y736/740/741F have an activated autophosphorylation activity even without pre-phosphorylation by Src. In the shc binding assay shown in figure 15, the mutant proteins of Y740F as well as Y736/740/741F showed an almost same behaviour with the wild type DDR2 cytosolic domain pre-phosphorylated by Src to give an enhanced she binding after autophosphorylation. This result suggests that Y740F mutation by itself is enough to mimic the action of Src to provide DDR2 cytosolic domain with enhanced shc binding as well as increased autophosphorylation activity. This results further supports the conclusion that Src activates DDR2 cytosolic signaling through the induction of tyrosine phosphorylation on tyrosine 740 residue. In addition, the fact that even the mutant protein of Y736/740/741F show the shc binding after autophosphorylation suggest that the three tyrosine residues in the activation loop of DDR2 do not participate in shc binding and other tyrosine residue phosphorylated by autophosphorylation is involved in the shc binding. Taken together these results demonstrate that the invented DDR2 cytosolic tyrosine kinase domain protein with i nduced tyrosine phosphorylation, for example, by the method of co-expression with c-Src or with fyn in sf 9 cells or by the H2O2 processing or with the mutation of tyrosine 740 to phenylalanine 740 can be used for in vitro shc binding assay and this binding assay can be more efficiently carried out by performing the in vitro autophosphorylation reaction.

The foregoing and other objects, features, aspects and advantages of the present invention will become more apparent from the following detailed description of the present invention when taken in conjunction with the accompanying drawings.

### Brief Description of the Drawings

The accompanying drawings, which are included to provide a further understa nding of the invention and are incorporated in and constitute a part of this specification, illustrate embodiments of the invention and together with the description serve to explain the principles of the invention.

In the drawings:

figure 1 shows the results determined by SDS-PAGE and a coomassie staining of the purified fused protein of Glutathione S Transferase and DDR2 cytosolic tyrosine kinase domain (GST-DDR2 CKD).

Figure 2 shows the results obtained by checking whether the tyrosines of the GST-DDR2 CKD were phosphorylated or not, through the western blotting using a phosphorylated tyrosine specific antibody after purification using glutathione bead; Lane 1: the case of solely expressing the GST-DDR2 CKD; Lane 2: the case of oo-expressing the GST-DDR2 CKD and a full-length c-Src protein; Lane 3: the case of co-expressing the GST-DDR2 CKD and a full-length c-Fyn protein; Lane 4: the case of solely expressing the GST-DDR2 CKD and then processing with H₂O₂ at the concentration of 200 µM for an hour. The presence of equal amount of protein at each lane was estimated by coomassie brilliant blue (CBB) staining. The co-expression of c-Src or c-Fyn protein was confirmed by western blotting on aliquot of total cell lysate using human c-Src or c-Fyn specific antibody.

Figure 3 shows that the tyrosine kinase activity of Src is indispensable to induce the tyrosine phosphorylation of DDR2 cytosolic tyrosine kinase domain when they are co-expressed in insect cells. GST-DDR2 CKD was expressed alone or co-expressed with c-Src or the kinase defective Src (kd-Src) in sf9 cells using baculoviral expression. The GST-DDR2 CKD was purified using glutathione bead and a equal amount of the protein, estimated by CBB staining, was subject to western blotting using P-tyro specific antibody.

Figure 4 shows that the full-length c-Src protein induces a tyrosine phosphorylation specifically to the DDR2 cytosolic tyrosine kinase domain. The results shown in Figure 4 have been obtained by co-expressing the full-length c-Src protein together with the fused proteins, GST-DDR2 CKD, GST-DDR1b CKD, GST-Akt1 and GST-CDK4, respectively; in sf9 cells, then performing a western blotting test on each purified GST tagged protein using a phosphorylated tyrosine specific antibody. The presence of a similar amount of each purified GST tagged protein was shown by CBB staining.

Figure 5 shows that H₂O₂ processing induces a tyrosine phosphorylation specifically to the DDR2 cytosolic tyrosine kinase domain. The results shown in Figure 5 have been obtained by solely expressing GST-DDR2 CKD, GST-DDR1b CKD, GST-CDK4 and GST-CDK1, respectively in sf9 cells; processing each expressed cells with H₂O₂ of 200 uM for half of an hour or not; and then purifying the GST tagged protein followed by a western blotting using a phosphorylated tyrosine specific antibody. The presence of a similar amount of each purified GST tagged protein was shown by CBB staining.

Figure 6 shows the results of determining the variation in the amount of the expressed GST-DDR2 CKD and the level of the tyrosine phosphorylation, depending on the combination ratio of GST-DDR2 CKD-coding baculoviral titer and c-Src-coding viral titer, when simultaneously transfecting sf9 cell with the baculovirus expressing the GST-DDR2 CKD and the baculovirus expressing the full-length c-Src protein with total MOI of 10 and purifying the GST-DDR2 CKD using glutathione bead. The amount of purified GST-DDR2 CKD was estimated by CBB staining and the degree of tyrosine phosphorylation was measured by western blotting using phospho-tyrosine specific antibody followed by chemiluminescence.

figure 7 shows the result of determining the level of the tyrosine phosphorylation in the glutathione bead purified GST-DDR2 CKD depending on the concentration of H ₂O₂, when processing the expressed sf9 cells with H₂O₂. The presence of the similar amount of each purified GST-DDR2 CKD was estimated by CBB staining and the degree of tyrosine phosphorylation was measured by western blotting using phospho-tyrosine specific antibody followed by chemiluminescence.

Figure 8 shows differences in the tyrosine kinase activities (measured using poly(D Y)n as a peptide substrate) as well as autophosphorylation activities among the GST-DDR2 CKD expressed alone in sf9 cells and purified using glutathione bead (GST-DDR2 CKD), GST-DDR2 CKD co-expressed with c-Src in sf9 cells and purified using glutathione bead (GST-DDR2 CKD + Src), GST-DDR2 CKD co-expressed with c-Fyn in sf9 cells and purified using glutathione bead (GST-DDR2 CKD + Fyn), and the GST-DDR2 CKD expressed alone in sf9 cells, then processed with 200 uM of H2O2 for 30 minutes, followed by purification using glutathione bead. A same amount of each purified GST-DDR2 CKD protein was used for each measurement.

figure 9 shows the variations of Km for ATP and Vmax determined by measuring a tyrosine kinase activities using 4 ug of Histone H2B as a substrate depending on the concentration of ATP and performing a reciprocal plotting of 1/V vs 1/[ATP(uM)] between GST-DDR2 CKD expressed alone in sf9 cells and purified using glutathione bead (GST-DDR2 CKD), GST-DDR2 CKD co-expressed with c-Src in sf9 cells and purified using glutathione bead (GST-DDR2 CKD + Src), The relative kinase activity for 10 minute reaction was regarded as a relative reaction velocity (V) because the activity showed a linearity in the course of 10 minute reaction. All measurements were done in triplicate.

Figure 10 shows the variations of Km for ATP and Vmax between GST-DDR2 CKD expressed alone in sf9 cells and purified using glutathione bead (GST-DDR2 CKD), the GST-DDR2 CKD expressed alone in sf9 cells, then processed with 200 uM of H2O2 for 30 minutes, followed by purification using glutathione bead.

figure 11 shows that the mutation in tyrosine 740 to phenylalanine 740 enhances the tyrosine phosphorylation in DDR2 cytosolic tyrosine kinase domain when expressed alone in sf9 cells. GST DDR2 CKD (alone or with Src) and each of its seven mutants described in the text were expressed in sf9 cells and purified using glutathione bead. A similar amount of each protein based on CBB staining was tested for a degree of tyrosine phosphorylation by western blotting using P-tyro antibody.

Figure 12 shows that the mutation of tyrosine 740 to phenylalanine 740 is enough to enhance the autophosphorylation activity of DDR2 cytosolic tyrosine kinase domain as much as Src did. The similar amounts of the purified wild and seven mutants of GST DDR2 CKD proteins were subject to in vitro autophosphorylation reaction in the presence of gamma-P³²-ATP as described in materials and methods. P³² radioactivities on the wild and mutant GST DDR2 CKD protein bands seen by CBB staining were visualized by autoradiography.

Figure 13 shows that the mutation of any tyrosine in the three tyrosine residues, 736, 740 and 741 can enhance the tyrosine kinase activity of DDR2 cytosolic tyrosine kinase domain independently of co-expression with Src GST DDR2 CKD and each of its seven mutants were expressed in sf9 cells alone (-) or with Src (+) and purified using glutathione bead. The same amount of each protein as judged by CBB staining was used to measure its in vitro tyrosine kinase activity using histone H2B as a substrate as described in materials and methods and their tyrosine kinase activities were shown by autoradiography. Negative stands for a kinase reaction without kinase protein as a control.

Figure 14 shows that Three tyrosine residues, 736, 740 and 741 in the activation loop of DDR2 are the targets for phosphorylation by Src. GST tagged kinase defective DDR2 cytosolic domain (GST-kd-DDR2-CKD) with intact three tyrosines in the activation loop or with various phenylalanine mutations described in the text were co-expressed with Src in sf9 cells. GST tagged proteins were purified and the same amount of proteins estimated by CBB staining was used for western blotting using P-tyro antibody.

Figure 15 shows that Autophosphorylation of DDR2 cytosolic domain is necessary for she binding to DDR2 cytosolic domain. The purified GST-DDR2 CKD proteins without or with tyrosine phosphorylation by Src were subject to in vitro she binding assay after autophosphorylation reaction or a mock-autophosphorylation reaction as described in materials and methods. The bound proteins on the glutathione bead were eluted by boiling in 1 x lameli buffer. The presence of she in the eluate was measured by western blotting using shc antibody and the accumulation of tyrosine phosphorylation at GST-DDR2 CKD proteins resulted from autophosphorylation was shown by western blotting using P-tyro antibody. The presence of an equal amount of GST-DDR2 CKD proteins in each eluate was confirmed by CBB staining. 'Bead only' means that no GST-DDR2 CKD protein was applied on glutathione bead as a control.

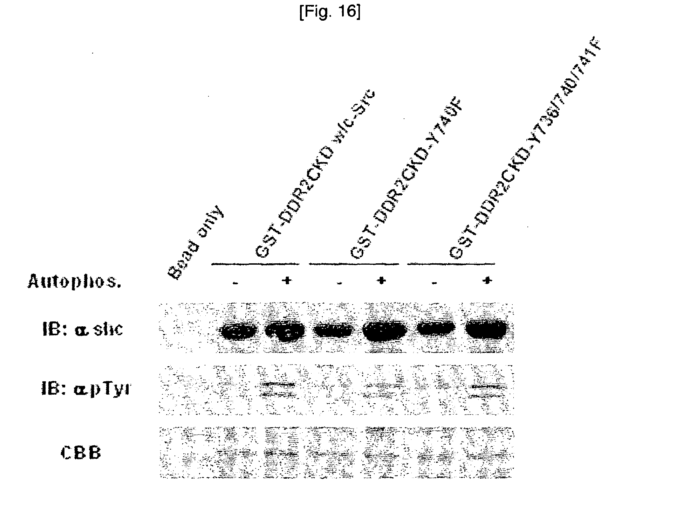

figure 16 shows that the mutation of tyrosine 740 to phenylalanine 740 enhances the shc binding after the autophosphorylation in DDR2 cytosolic tyrosine kinase domain. The same type of experiment was done as figure 15 using purified GST - DDR2 CKD phosphorylated by src and its mutant proteins of Y740F and Y736/740/741F.

### Mode for the Invention

The present invention will now be described in detail with reference to preferred embodiments as follows. However, the present invention may not be limited by those preferred embodiments.

Materials and Methods

*Construction of plasmids*

All baculoviral expression vectors were constructed using pBacPAK8 vector (Clontech, U.S.A). For a convenient tagging of glutathione S transferase (GST) gene to expressed proteins, pBacPAK8 vector was modified to contain GST gene by subcloning pcr'ed c-DNA fragment of GST from pGEX4T1 plasmid (cat #: 27-4580-01, Amersham Biosciences, U.S.A) having Bgl II restriction site at 5' position and Bam HI or Xho I site at 3' position into Bam HI or Bam H-Xho I cutting sites in pBacPAK8 plasmid (pBacPak 8-GST-Bam HI or pBacPak 8-GST-Xho I). For the baculoviral expression vector of GST tagged DDR2 cytosolic tyrosine kinase domain (GST-DDR2-CKD), pcr amplified c-DNA fragment covering human DDR2 from amino acids 441 to 815 using 5' primer of ccc gga tac atg aca gtc agc ctt tcc ct (SEQ ID NO: 7) and 3' primer of ggg tct aga tca ctc gtc gcc ttg ttg aag (SEQ ID NO: 7) was subcloned into Xho I-Not I site of pBacPAK8-GST-Xho I vector.

Baculoviral GST tagged CDK4 (GST-CDK4) expression vector was made by subcloning pcr amplified full length human CDK4 gene into Bam H I-Not I site of pBacRAK 8-GST-Bam H I vector. GST tagged DDR1 cytosolic tyrosine kinase domain (GST-DDR1-CKD) expression vector was made by subcloning of pcr amplified kinase domain gene of human DDR1b (amino acids from 454 to 914) into Bam H I-Not I site of pBacPAK 8-GST-Bam H1 vector. GST tagged mouse Akt1 (GST-Aktl) expression vector was prepared by subcloning the pcr amplified full length c-DNA of mouse Akt1 gene into Bam H1 -Not1 site of pBacPAK 8-GST-Bam H1 vector.

Full length human c-Src gene was pcr amplified from the vector of pUSE human c-Src wild (Upstate Biotechnology) using 5' primer of GGGGGATTCGACG-GATCGGGAGATCTCCCG (SEQ ID NO: 8) and 3' primer of CCCGAATTCGAC GTC AGG TGG CAC TTT TCG GGG (SEQ ID NO: 9), and was subcloned into Xho I-Eco R1 site of pBacPAK 8 vector without any tagging. Kinase inactive c-Src gene was pcr amplified from the vector of pUSE human c-Src negative (Upstate Biotechnology) and was subcloned into Xho 1-Eco R1 site of pBacPAK 8 vector without any tagging. Full length human c-Fyn gene was pcr amplified from the vector containing full length human c-Fyn urchased from Invitrogen, U.S.A. and subcloned into Xho I-Eco R1 site of pBacPAK 8 vector without any tagging. The sequence correctness of all per amplified DNAs was verified by extensive sequencing.

*Site directed mutagenesis*

Site directed mutagenesis in pBacPAK8-GST-DDR2 CKD was performed using PCR technique. Briefly, to generate a baculoviral expression vector of GST tagged kinase defective DDR2 kinase domain (pBacPAK8-GST-kd-DDR2 CKD), lysine 608 to alanine 608 mutation was introduced into GST-DDR2 cytosolic domain by replacing Nco I-Bam HI fragment within DDR2 cytosolic domain gene with a pcr'ed fragment to contain the mutation of K608A from overlapping pcr technique using four primers of gccgtcaccatggacctg (forward primer containing Nco I site, SEQ ID NO: 10), gcccggccctggatccgg (reverse primer containing Bam HI site, SEQ ID NO: 11), gtggctgtggcaatgctccga (forward primer containing K608A mutation, SEQ ID NO: 12), and tcggagcattgccacagccac (reverse primer containing K608A mutation, SEQ ID NO: 13).

To introduce site directed mutations on the three tyrosine residues (Y736, Y740, and Y741) in the activation segment region of wild type DDR2 tyrosine kinase domain or kinase defective DDR2 tyrosine kinase domain, we pcr-amplified fourteen NcoI/ BamHI fragments of seven from DDR2 CKD and seven from kinase defective DDR2 CKD(K608A) c-DNA using seven sets of primer pairs consisting of 5' primer (actcagtgcctgccgtcacc, SEQ ID NO: 14) containing Nco I site and each of seven different 3' primers containing each mutation as well as BamHI site such as Y740F primer (cccggccctggatccggtaatagtcaccactgaacaggttc, SEQ ID NO: 15), Y736F primer, (cccggccctggatccggtaaaagtcacc, SEQ ID NO: 16), Y471F primer (cccggccctggatccggaaatagtc, SEQ ID NO: 17), Y736, 740F primer (cccggccctggatccggtaaaagtcaccactgaacaggttg, SEQ ID NO: 18), Y736,741F primer (cccggccctggatccggaaatagtcaccactgaacaggtc, SEQ ID NO: 19), Y740,741F primer (cccggccctggatccggaaaaagtcacc, SEQ ID NO: 20), and Y736,740,741F primer (cccggccctggatccggaaaaagtcaccactgaacaggttcc, SEQ ID NO: 21), respectively. Each of fourteen pcr'ed fragments was subcloned into NcoI/BamHI site in pBacPAK8-GST-DDR2 CKD.

*Cell lines and culture*

*Spodoptera frugoperda* SF9 cells were maintained in TNM-FH insect medium (Sigma) supplemented with 10% heat-inactivated fetal bovine serum, 50ug/ml gentamyan (Sigma), and 2 mM glutamine (Gibco-BRL) at 27°C. HSC-T6, a human hepatostellate cell line was cultured in Dulbeaoo's modified eagle medium (DMEM) in presence of 10% fetal bovine serum, 150ug/ml penicillin-streptomycin (Gibco-BRL), under 5% CO at 37°C

*Baculoviral expressions and purificaton of proteins.*

Each generated baculoviral expression vector plasmid DNA was transfected into sf9 cells along with viral genomic DNA from baculovirus generation kit purchased from Clontech according to the manufacturer's manual. The viral stock was amplified to have a titer of about 10⁸ pfu /ml. The virus were infected into sf9 cells with MOI 10 and left for 48 hours before harvest of cells. GST tagged DDR2 c-terminal kinase domain proteins were purified using glutathione agarose bead affinity column and subsequent Superdex 200 prep grade gel filtration (Amersham) using FPLC system.

Total 400 ml culture of sf 9 cells after two days of virus infection were detached from ten T150 culture dishes using glass capillary pipet and harvested by centrifugation at 1500 rpm for 5 minutes and briefly rinsed using 1 x TBS. Cells were suspended in 20 ml of lysis buffer consisting of 20mM Tris-HCl (pH8.0), 0.15 M NaCl, 1mM DTT, 10mM NaF, 0.1mM EDTA, 0.1M sodium vanadate, 0.02% IGE-PAL (Sigma), a proteinase inhibitor cocktail pellet (Roche) and lysed by sonication for 1 min. The lysate was centrifuged at 12,000 rpm for 30 min using Sorval SS34 rotor (Beckmann) and the supernatant was applied to 1 ml bed volumn of glutathione agarose bead column (Amersham) pre-equilibrated with 20mM Tris-HCl (pH8.0), 0.15 M NaCl buffer and washed with the column equilibration buffer.

The bound proteins were eluted with 10 ml of washing buffer containing 20 mM reduced glutathione and subsequently concentrated to 1 ml using Vivaspin concentrator. For a brief purification of GST tagged proteins bound to glutathione bead, infected sf9 cells of 4 ml culture were detached from T25 culture plate and rinsed with 1xTBS. Cells were lysed in 500 ul of the lysis buffer described above by sonication and centrifuged at 12,000 rpm using microfuge for 10 min. 50 ul of 50% slurry of glutathione bead was added to the supernatent and slowly rotated for 10 min at 4°C. Finally the GST tagged protein bound bead was obtained by washing with 1 x TBS for three times.

*Induction of tyrosine phosphorylation at DDR2 cytosolic tyrosine kinase domain by H₂O*₂ *processing*

The recombinant baculoviruses having the GST-DDR2 CKD coding gene were transfected into sf9 insect cells, by setting MOU to 10 and the cells were cultured for 24 to 72 hours, to express the protein. H₂O₂ at the concentration of 200 µM was added to the cultured cells. The cells were left for half of an hour, and collected. The collected cells were lysed and the GST-DDR2 CKD was purified from the expressed products, using the glutathione agarose bead column chromatography. The purified protein was subjected to an electrophoresis in 10 % polyacrylamaid gel.

After completing the electrophoresis, the protein developed in the gel was transferred to nitrocellulose, and then subjected to a western blotting using a phosphorylated tyrosine specific antibody (Cell signaling Inc, USA), to measure the chemiluminescence signal using x-ray film. For the control, the chemical luminescence of the case without H₂O₂ processing was measured by the method as mentioned above.

Similarly to the GST-DDR2 CKD protein, the tyrosine phosphorylation was also been induced in each fused protein with GST of DDR1b cytosolic tyrosine kinase domain, Akt1 and CDK4 through processing with H₂O₂.

*Measurements for autophosphorylation and tyrosine kinase activity of DDR2 cytosolic tyrosine kinase.*

For measuring the autophosphorylation activity, the reaction was performed using 100-300 ng of DDR2 cytosolic domain protein in 20 ul of reaction mixture containing 20 mM Tris-HCl, pH 8.0, 5 mM MgCl₂, 0.5 mM dithiothreitol, 0.01 mM ATP and 0.2 uCi of P³²-ATP. After 15 min incubation at 30°C, the reaction was stopped by adding a half volume of 3x lameli with subsequent boiling for 2 minutes. The stopped mixture was run in 10% SDS-PAGE gel and the portion of gel containing unreacted free ATP was removed and the remaining gel was stained using coomagie brilliant blue (Sigma) and dried. P³² radioactivity in the stained DDR2 cytosolic kinase protein band was visualized by autoradiography and quantitated using BAS P³²-image analyzer.

Measurement of DDR2 tyrosine kinase activity towards heterologous peptide substrates was performed using 100 ng of the purified kinase protein in 20 ul of reaction mixture containing 20 mM Tris-HCl, pH 8.0, 5 mM MgCl₂, 0.5 mM dithiothreitol, 0.01 mM ATP. 4ug of peptide substrate such as histone H2B (Sigma) or poly(D₄Y)n (Promega) and 0.2 uCi of P³²-ATP. After 15 min incubation at 30°C, the reaction was stopped by adding a half volume of 30% phosphoric add. When H2B was used as a peptide substrate, the reaction mixture was spotted on p81 paper (Millipore). For poly(D₄Y)n as a substrate, it was spotted on avidine coated membrane (Promega). The spotted filter was washed with 0.1M Tris-HCl (pH 8.0) five times for 10 min each and the radioactivity of each spot was visualized and quantitated using BAS image analyzer (Kodak).

*Antibodies and immunoblotting*

Phospho-tyrosine specific antiboby was purchased from Cell Signaling Inc, US.A. Specific antibodies to human c-Src, human c-Fyn, and she were obtained from SantaCruz, US.A. Samples were boiled for 2min in 1x lameli sample buffer, and loaded in 10% SDS-PAGE Proteins were transferred to Immobilon-P membrane (Millipore) and blocked by 10% skim milk in TBS for an hour. The membrane was immunoblotted with antibodies for 2 hours and subsequently washed with 1xTBS five times. Then it was incubated with HRP conjugated secondary antibodies for an hour and protein bands were detected using chemiluminescence kit (Amersham).

*Shc binding assay*

As a source of she protein, total lysate of HSC T6 cells was used. For the preparation of the lysate, 2 x 10⁷ HSC T6 cells were harvested and lysed by sonication in 5 ml of lysis buffer consisting of 20mM Tris-HCl (pH8.0), 0.1M NaCl, 5mM DTT, 10mM NaF, 0.1M orthovanadate, 0.02% IGE-PAL (Sigma) and a proteinase inhibitor cocktail pellet (Roche). The supernatant was obtained by centrifugation in 15,000 rpm for 10min at 4°C and used for the following she binding assay. Various GST-DDR2 KD proteins bound to glutathione bead were obtained as described above.

For the preparation of autophosphorylated GST-DDR2 CKD proteins bound to the bead, kinase reaction mixture containing Tris-HCl (pH 7.5), 0.1M NaCl, 5 mM MgCl₂, 1 mM DTT and 100 uM of ATP was added to the protein bound glutathione bead and incubated at 30°C for 15 min, and then washed three times with cold 1xTBS. Mock-autophosphorylation was carried using gamma-S-ATP instead of ATP. As the shc binding step, HSC T6 cell lysate was added to the protein bound bead and slowly rotated for an hour at 4°C. Next, the bead was washed with cold 1xTBS for three times and the bound proteins were eluted by boiling the bead at 1x lameli buffer for 2 min. The eluted proteins were applied to 10% SDS-PAGE and performed a western blotting using the monoclonal antiboby specific to she to examine the presence of bound shc protein as well as coomagie brilliant blue dye staining to confirm the presence of equal amount of GST-DDR2 CKD protein at each bead preparation.

### Industrial Applicability

Therefore, the present invention relating to a method for preparing the protein having increased DDR2 autophosphorylation and tyrosine kinase activity in a large amount and the protein containing the DDR2 cytosolic tyrosine kinase domain having increased autophosphorylation and tyrosine kinase activity thereby, may be useful as a target protein for discovering a medical drug in developing new drugs for treating the diseases caused by the excessive DDR2 autophosphorylation and tyrosine kinase activity, especially the diseases mainly caused by the growth of a fibrotic cells, such as liver cirrhosis, arteriosclerosis, rheumatism and the like, as well as cancer.

## Claims

1. A protein containing a modified human DDR2 cytosolic tyrosine kinase domain having an increased autophosphorylation and tyrosine kinase activity, wherein tyrosine 740 in the activation loop of the human DDR2 cytosolic tyrosine kinase domain is modified by inducing a phophorylation of tyrosine 740, or by mutating to phenylalanine 740 by a site-directed mutation.

2. Method for preparing a protein containing a human DDR2 cytosolic tyrosine kinase domain having increased autophosphorylation and tyrosine kinase activity, through phosphorylation of tyrosine 740 at the DDR2 cytosolic tyrosine kinase domain, comprising the following steps of:
- amplifying DNA fragment which encodes an amino acid sequence sufficiently covering a DDR2 cytosolic tyrosine kinase domain protein, and introducing the amplified D NA fragment into a viral expression vector to construct a recombinant viral expression vector for DDR2 cytosolic tyrosine kinase domain protein and generating recombinant virus carrying the DDR2 cytosolic tyrosine kinase domain protein;
- amplifying DNA fragment which encodes an amino acid sequence sufficiently covering a full-length c-Src or c-Src related protein, and introducing the amplified DNA fragment into another separate virus expression vector genome, to construct a recombinant virus expression vector for the c-Src or c-Src related protein and generating recombinant virus carrying the c-Sre or c-Src related protein;
- infecting the obtained virus carrying the DDR2 cytosolic tyrosine kinase domain and the obtained virus carrying the c-Src or c-Src related protein into a host cell, co-expressing the proteins together, and inducing a tyrosine phosphorylation at the DDR2 cytosolic tyrosine kinase domain by the tyrosine kinase activity of c-Src or c-Src related protein, to produce a large amount of a protein containing the DDR2 cytosolic tyrosine kinase domain with increased tyrosine phosphorylation;
- isolating and purifying the obtained protein containing the DDR2 cytosolic tyrosine kinase domain with increased tyrosine phosphorylation, wherein tyrosine 740 of the DDR2 cytosolic tyrosine kinase domain is essentially phosphorylated,
wherein the virus is a baculovirus and the host cell is an insect cell.

3. The method of claim 2, wherein the c-Src related protein is selected from the group consisting of v-Src, Fyn, yes, Lck, Hck, Csk and Blk including their tyrosine kinase-activated versions.

4. The method of claim 2, wherein the DDR2 cytosolic tyrosine kinase domain protein is tagged with one selected from the group consisting of glutathione-S-transferase, thioredoxin or histidine oligomer.

5. The method of claim 2, wherein the virus carrying the DDR2 cytosolic tyrosine kinase domain protein and the virus carrying the o-Src or c-Src related protein are simultaneously infected into the host cell at the combination ratio of 1:3 to 3:1 and the MOI (multiplicity of infection) of I to 10.

6. A method for preparing a protein containing a human DDR2 cytosolic tyrosine kinase domain having an increased autophosphorylation and tyrosine kinase activity, by phosphorylating tyrosine 740 at the DDR2 cytosolic tyrosine kinase domain protein, comprising the following steps of:
- amplifying DNA fragment which encodes an amino acid sequence sufficiently covering a DDR2 cytosolic tyrosine kinase domain protein, and introducing the amplified DNA fragment into a viral expression vector to construct a recombinant viral expression vector for DDR2 cytosolic tyrosine kinase domain protein and generating recombinant virus carrying the DDR2 cytosolic tyrosine kinase domain protein;
- infecting the obtained the virus of the DDR2 cytosolic tyrosine kinase domain into a host cell, to produce a protein containing the DDR2 cytosolic tyrosine kinase domain, and then treating the cells with H₂O₂ at the concentration of 10 µM to 1 mM to induce tyrosine phosphorylation at the expressed DDR2 cytosolic tyrosine kinase domain; and
- isolating and purifying the expressed protein containing the DDR2 cytosolic tyrosine kinase domain with induced tyrosine phosphorylation, wherein tyrosine 740 of the DDR2 cytosolic tyrosine kinase domain is essentially phosphorylated, wherein the virus is a baculovirus and the host cell is an insect cell.

7. The method of claim 6, wherein the DDr2 cytosolic tyrosine kinase domain protein is tagged with one selected from the group consisting of glutathione-S-transferase, thioredoxin or histidine oligomer.

8. A method for preparing a protein containing a human DDR2 cytosolic tyrosine kinase domain having an increased autophosphorylation and tyrosine kinase activity, by mutating tyrosine 740 at the DDR2 cytosolic tyrosine kinase domain, comprising the following steps of:
- amplifying DNA fragment which encodes an amino acid sequence sufficiently covering a DDR2 cytosolic tyrosine kinase domain protein where tyrosine 740 at the DDR2 cytosolic tyrosine kinase domain is mutated to phenylalanine by a site-directed mutagenesis, and introducing the amplified DNA fragment into a viral expression vector to construct a recombinant viral expression vector for DDR2 cytosolic tyrosine kinase domain with mutation of tyrosine 740 to phenylalanine 740, and generating recombinant virus carrying the mutant DDR2 cytosolic tyrosine kinase domain;
- infecting the obtained recombinant virus of the mutant DDR2 cytosolic tyrosine kinase domain into a host cell, to produce a protein containing the mutant DDR2 cytosolic tyrosine kinase domain,
- isolating and purifying the expressed mutant protein containing the DDR2 cytosolic tyrosine kinase domain with mutation of tyrosine 740 to phenylalanine 740.
wherein the virus is a baculovirus and the host cell is an insect cell.

9. The method of claim 8, wherein the DDR2 cytosolic tyrosine kinase domain protein is tagged with one selected from the group consisting of glutathione-S-transferase, thioredoxin or histidine oligomer.

10. An in vitro use of a protein containing a modified DDR2 cytosolic tyrosine kinase domain having an increased autophosphorylation and tyrosine kinase activity, to be utilized in the development of medical drugs for treating a disease caused by an excessive autophoshorylation and tyrosine kinase activity of DDR2 protein, wherein tyrosine 740 of the activation loop of human DDR2 cytosolic tyrosine kinase domain is modified by including a phosphorylation of tyrosine 740, or by mutating to phenylalanine 740 by a site-directed mutation.

## Patentansprüche

1. Protein, enthaltend eine modifizierte humane zytosolische DDR2-Tyrosinkinase-Domäne mit einer erhöhten Autophosphorylierungs- und Tyrosinkinase-Aktivität, bei dem Tyrosin 740 in der Aktivierungsschleife der humanen zytosolischen DDR2-Tyrosinkinase-Domäne durch Einführen einer Phosphorylierung von Tyrosin 740 oder durch Mutieren zu Phenylalanin 740 durch eine ortsspezifische Mutation modifiziert ist.

2. Verfahren zur Herstellung eines Proteins, das eine humane zytosolische DDR2-Tyrosinkinase-Domäne mit einer erhöhten Autophosphorylierungs- und Tyrosinkinase-Aktivität enthält, durch Phosphorylierung von Tyrosin 740 in der zytosolischen DDR2-Tyrosinkinase-Domäne, umfassend die folgenden Schritte:
- Amplifizieren eines DNA-Fragments, das eine Aminosäuresequenz codiert, welche ausreichend ein zytosolische DDR2-Tyrosinkinase-Domäne-Protein abdeckt, und Einführen des amplifizierten DNA-Fragments in einen viralen Expressionsvektor, um einen rekombinanten viralen Expressionsvektor für zytosolische DDR2-Tyrosinkinase-Domäne-Protein zu konstruieren, und Erzeugen eines rekombinanten Virus, welches das zytosolische DDR2-Tyrosinkinase-Domäne-Protein trägt;
- Amplifizieren eines DNA-Fragments, das eine Aminosäuresequenz codiert, die ausreichend ein c-Src- oder mit c-Src verwandtes Protein in voller Länge abdeckt, und Einführen des amplifizierten DNA-Fragments in ein anderes getrenntes Virus-Expressionsvektor-Genom, um einen rekombinanten Virusexpressionsvektor für das c-Src- oder mit c-Src verwandte Protein zu konstruieren, und Erzeugen eines rekombinanten Virus, der das c-Src- oder das mit c-Src verwandte Protein trägt;
- Einschleusen des erhaltenen Virus, welches die zytosolische DDR2-Tyrosinkinase-Domäne trägt, und des erhaltenen Virus, weiches das c-Src- oder mit c-Src verwandte Protein trägt, in eine Wirtszelle durch Infektion, Coexprimieren der Proteine zusammen und Induzieren einer Tyrosin-Phosphorylierung an der zytosolischen DDR2-Tyrosinkinase-Domäne durch die Tyrosinkinase-Aktivität des c-Src- oder mit c-Src verwandten Proteins, um eine große Menge eines Proteins zu produzieren, welches die zytosolische DDR2-Tyrosinkinase-Domäne mit erhöhter Tyrosin-Phosphorylierung enthält;
- Isolieren und Reinigen des erhaltenen Proteins, welches die zytosolische DDR2-Tyrosinkinase-Domäne mit erhöhter Tyrosin-Phosphorylierung enthält, wobei Tyrosin 740 der zytosolischen DDR2-Tyrasinkinase-Domäne im Wesentlichen phosphoryliert ist,
wobei das Virus ein Baculovirus ist und die Wirtszelle eine Insektenzelle ist.

3. Verfahren nach Anspruch 2, bei dem das mit c-Src verwandte Protein ausgewählt ist aus der Gruppe bestehend aus v-Src, Fyn, yes, Lck , Hck, Csk und Blk, einschließlich ihrer Tyrosinkinase-aktivierten Versionen.

4. Verfahren nach Anspruch 2, bei dem das zytosolische DDR2-Tyrosinkinase-Domäne-Protein mit einem Mitglied markiert ist, das ausgewählt ist aus der Gruppe bestehend aus Glutathion-S-Transferase, Thioredoxin oder Histidin-Oligomer.

5. Verfahren nach Anspruch 2, bei dem das Virus, welches das zytosolische DDR2-Tyrosinkinase-Domäne-Protein trägt, und das Virus, welches das c-Src- oder mit c-Src verwandte Protein trägt, gleichzeitig mit einem Kombinationsverhältnis von 1:3 bis 3:1 und einer MOI (Multiplizität der Infektion) von 1 bis 10 in die Wirtszelle durch Infektion eingeschleust werden.

6. Verfahren zur Herstellung eines Proteins, das eine humane zytosolische DDR2-Tyrosinkinase-Domäne mit einer erhöhten Autophosphorylierungs- und Tyrosinkinase-Aktivität enthält, durch Phosphorylierung von Tyrosin 740 in dem zytosolische DDR2-Tyrosinkinase-Domäne-Protein, umfassend die folgenden Schritte:
- Amplifizieren eines DNA-Fragments, das eine Aminosäuresequenz codiert, welche ausreichend ein zytosolische DDR2-Tyrosinkinase-Domäne-Protein abdeckt, und Einführen des amplifizierten DNA-Fragments in einen viralen Expressionsvektor, um einen rekombinanten viralen Expressionsvektor für das zytosolische DDR2-Tyrosinkinase-Domäne-Protein zu konstruieren, und Erzeugen eines rekombinanten Virus, welches das zytosolische DDR2-Tyrosinkinase-Domäne-Protein trägt;
- Einschleusen des erhaltenen Virus der zytosolischen DDR2-Tyrosinkinase-Domäne in eine Wirtszelle durch Infektion, um ein Protein zu produzieren, welches die zytosolische DDR2-Tyrosinkinase-Domäne enthält, und anschließendes Behandeln der Zellen mit H₂O₂ in einer Konzentration von 10 µM bis 1 mM, um die Tyrosin-Phosphorylierung an der exprimierten zytosolischen DDR2-Tyrosinkinase-Domäne zu induzieren; und
- Isolieren und Reinigen des exprimierten Proteins, das die zytosolische DDR2-Tyrosinkinase-Domäne mit induzierter Tyrosin-Phosphorylierung enthält, wobei Tyrosin 740 der zytosolischen DDR2-Tyrosinkinase-Domäne im Wesentlichen phosphoryliert ist, wobei das Virus ein Baculovirus ist und die Wirtszelle eine Insektenzelle ist.

7. Verfahren nach Anspruch 6, bei dem das zytosolische DDR2-Tyrosinkinase-Domäne-Protein mit einem Mitglied markiert ist, das ausgewählt ist aus der Gruppe bestehend aus Glutathion-S-Transferase, Thioredoxin oder Histidin-Oligomer.

8. Verfahren zur Herstellung eines Proteins, das eine humane zytosolische DDR2-Tyrosinkinase-Domäne mit einer erhöhten Autophosphorylierungs- und Tyrosinkinase-Aktivität enthält, durch Mutieren von Tyrosin 740 in der zytosolischen DDR2-Tyrosinkinase-Domäne, umfassend die folgenden Schritte:
- Amplifizieren eines DNA-Fragments, das eine Aminosäuresequenz codiert, die ausreichend ein zytosolische DDR2-Tyrosinkinase-Domäne-Protein abdeckt, wobei Tyrosin 740 an der zytosolischen DDR2-Tyrosinkinase-Domäne durch eine ortsspezifische Mutagenese zu Phenylalanin mutiert ist, und Einführen des amplifizierten DNA-Fragments in einen viralen Expressionsvektor, um einen rekombinanten viralen Expressionsvektor für die zytosolische DDR2-Tyrosinkinase-Domäne mit einer Mutation von Tyrosin 740 zu Phenylalanin 740 zu konstruieren, und Erzeugen eines rekombinanten Virus, das die mutierte zytosolische DDR2-Tyrosinkinase-Domäne trägt;
- Einschleusen des erhaltenen rekombinanten Virus der mutierten zytosolischen DDR2-Tyrosinkinase-Domäne in eine Wirtszelle durch Infektion, um ein Protein zu produzieren, das die mutierte zytosolische DDR2-Tyrosinkinase-Domäne enthält,
- Isolieren und Reinigen des exprimierten mutierten Proteins, das die zytosolische DDR2-Tyrosinkinase-Domäne mit einer Mutation von Tyrosin 740 zu Phenylalanin 740 enthält,
wobei das Virus ein Baculovirus ist und die Wirtszelle eine Insektenzelle ist.

9. Verfahren nach Anspruch 8, bei dem das zytosolische DDR2-Tyrosinkinase-Domäne-Protein mit einem Mitglied markiert ist, das ausgewählt ist aus der Gruppe bestehend aus Glutathion-S-Transferase, Thioredoxin oder Histidin-Oligomer.

10. In-vitro-Verwendung eines Proteins, das eine modifizierte zytosolische DDR2-Tyrosinkinase-Domäne mit einer erhöhten Autophosphorylierungs- und Tyrosinkinase-Aktivität enthält, zur Verwendung bei der Entwicklung von medizinischen Arzneistoffen zur Behandlung einer Krankheit, die durch eine übermäßige Autophosphorylierungs- und Tyrosinkinase-Aktivität von DDR2-Protein verursacht wird, wobei Tyrosin 740 der Aktivierungsschleife der humanen zytosolischen DDR2-Tyrosinkinase-Domäne durch Einschließen einer Phosphorylierung von Tyrosin 740 oder durch Mutieren zu Phenylalanin 740 durch eine ortsspezifische Mutation modifiziert ist.

## Revendications

1. Protéine contenant un domaine modifié de la tyrosine kinase DDR2 cytosolique humaine ayant une activité d'autophosphorylation et de tyrosine kinase augmentée, dans laquelle la tyrosine 740 dans la boucle d'activation du domaine de la tyrosine kinase DDR2 cytosolique humaine est modifiée par induction d'une phosphorylation de la tyrosine 740 ou par mutation en phénylalanine 740 par une mutation dirigée sur un site.

2. Procédé pour la préparation d'une protéine contenant un domaine de la tyrosine kinase DDR2 cytosolique humaine ayant une activité d'autophosphorylation et de tyrosine kinase augmentée par phosphorylation de la tyrosine 740 au niveau du domaine de la tyrosine kinase DDR2 cytosolique, comprenant les étapes suivantes :
- amplification du fragment d'ADN qui code une séquence d'acides aminés couvrant suffisamment une protéine du domaine de la tyrosine kinase DDR2 cytosolique et introduction du fragment d'ADN amplifié dans un vecteur d'expression viral pour construire un vecteur d'expression viral de recombinaison pour la protéine du domaine de la tyrosine kinase DDR2 cytosolique et génération d'un virus de recombinaison portant la protéine du domaine de la tyrosine kinase DDR2 cytosolique,
- amplification du fragment d'ADN qui code une séquence d'acides aminés couvrant suffisamment une protéine c-Src ou apparentée à c-Src en pleine longueur et introduction du fragment d'ADN amplifié dans le génome d'un autre vecteur d'expression viral séparé pour construire un vecteur d'expression viral de recombinaison pour la protéine c-Src ou apparentée à c-Src et génération d'un virus de recombinaison portant la protéine c-Src ou apparentée à c-Src,
- infection avec le virus obtenu portant le domaine de la tyrosine kinase DDR2 cytosolique et le virus obtenu portant la protéine c-Src ou apparentée à c-Src d'une cellule hôte co-exprimant les deux protéines et inclusion d'une phosphorylation de la tyrosine dans le domaine de la tyrosine kinase DDR2 cytosolique par l'activité tyrosine kinase de la protéine c-Src ou apparentée à c-Src, pour produire une grande quantité de protéine contenant le domaine de la tyrosine kinase DDR2 cytosolique à phosphorylation accrue de la tyrosine,
- isolation et purification de la protéine obtenue contenant le domaine de la tyrosine kinase DDR2 cytosolique à phosphorylation accrue de la tyrosine, dans laquelle la tyrosine 740 du domaine de la tyrosine kinase DDR2 cytosolique est pour l'essentiel phosphorylée,
dans lequel le virus est un baculovirus et la cellule hôte est une cellule d'insecte.

3. Procédé selon la revendication 2, dans lequel la protéine apparentée à c-Src est sélectionnée parmi v-Src, Fyn, yes, Lek, Hck, Csk et Blk, y compris leurs versions activées par la tyrosine kinase.

4. Procédé selon la revendication 2, dans lequel la protéine du domaine de la tyrosine kinase DDR2 cytosolique est marquée avec de la glutathione-S-transférase, de la thiorédoxine ou un oligomère d'histidine.

5. Procédé selon la revendication 2, dans lequel le virus portant la protéine du domaine de la tyrosine kinase DDR2 cytosolique et le virus portant la protéine c-Src ou apparentée à c-Src sont utilisés pour infecter simultanément la cellule hôte dans une proportion de 1 pour 3 à 3 pour 1 et la multiplicité d'infection (MOI) est de 1 à 10.

6. Procédé pour la préparation d'une protéine contenant un domaine modifié de la tyrosine kinase DDR2 cytosolique humaine ayant une activité d'autophosphorylation et de tyrosine kinase augmentée par phosphorylation de la tyrosine 740 au niveau du domaine de la tyrosine kinase DDR2 cytosolique, comprenant les étapes suivantes :
- amplification du fragment d'ADN qui code une séquence d'acides aminés couvrant suffisamment une protéine du domaine de la tyrosine kinase DDR2 cytosolique et introduction du fragment d'ADN amplifié dans un vecteur d'expression viral pour construire un vecteur d'expression viral de recombinaison pour la protéine du domaine de la tyrosine kinase DDR2 cytosolique et génération d'un virus de recombinaison portant la protéine du domaine de la tyrosine kinase DDR2 cytosolique,
- infection d'une cellule hôte avec le virus obtenu portant le domaine de la tyrosine kinase DDR2 cytosolique pour produire une protéine contenant le domaine de la tyrosine kinase DDR2 cytosolique, puis traitement des cellules avec H₂O₂ à une concentration de 10 µM à 1 mM pour induire la phosphorylation de la tyrosine dans le domaine de la tyrosine kinase DDR2 cytosolique exprimé ; et
- isolation et purification de la protéine exprimée contenant le domaine de la tyrosine kinase DDR2 cytosolique à phosphorylation induite de la tyrosine, dans laquelle la tyrosine 740 du domaine de la tyrosine kinase DDR2 cytosolique est pour l'essentiel phosphorylée,
dans lequel le virus est un baculovirus et la cellule hôte une cellule d'insecte.

7. Procédé selon la revendication 6, dans lequel la protéine de domaine de la tyrosine kinase DDR2 cytosolique est marquée avec de la glutathione-S-transférase, de la thiorédoxine ou un oligomère d'histidine.

8. Procédé pour la préparation d'une protéine contenant un domaine modifié de la tyrosine kinase DDR2 cytosolique humaine ayant une activité d'autophosphorylation et de tyrosine kinase augmentée par phosphorylation de la tyrosine 740 au niveau du domaine de la tyrosine kinase DDR2 cytosolique, comprenant les étapes suivantes :
- amplification du fragment d'ADN qui code une séquence d'acides aminés couvrant suffisamment une protéine du domaine de la tyrosine kinase DDR2 cytosolique, la tyrosine 740 du domaine de la tyrosine kinase DDR2 cytosolique étant mutée en phénylalanine par mutagenèse dirigée sur un site, et introduction du fragment d'ADN amplifié dans un vecteur d'expression viral pour construire un vecteur d'expression viral de recombinaison pour le domaine de la tyrosine kinase DDR2 cytosolique avec une mutation de la tyrosine 740 en phénylalanine 740, et génération d'un virus de recombinaison portant le domaine de la tyrosine kinase DDR2 cytosolique mutant,
- infection d'une cellule hôte avec le virus de recombinaison obtenu porteur du domaine de la tyrosine kinase DDR2 cytosolique mutant pour produire une protéine contenant le domaine de la tyrosine kinase DDR2 cytosolique mutant,
- isolation et purification de la protéine mutante exprimée contenant le domaine de la tyrosine kinase DDR2 cytosolique à mutation de la tyrosine 740 en phénylalanine 740,
dans lequel le virus est un baculovirus et la cellule hôte est une cellule d'insecte.

9. Procédé selon la revendication 8, dans lequel la protéine du domaine de la tyrosine kinase DDR2 cytosolique est marquée avec de la glutathione-S-transférase, de la thiorédoxine ou un oligomère d'histidine.

10. Utilisation in vitro d'une protéine contenant un domaine modifié de la tyrosine kinase DDR2 cytosolique ayant une activité d'autophosphorylation et de tyrosine kinase accrue, destiné à être utilisée dans le développement de médicaments pour le traitement d'une maladie causée par une activité d'autophosphorylation et de tyrosine kinase excessive de la protéine DDR2, dans laquelle la tyrosine 740 de la boucle d'activation du domaine de la tyrosine kinase DDR2 cytosolique humaine est modifiée par induction d'une phosphorylation de la tyrosine 740 ou par mutation en phénylalanine 740 par une mutation dirigée sur un site.
